(19) **Europäisches Patentamt / European Patent Office / Office européen des brevets**

(11) **EP 4 566 609 A1**

(12) **EUROPEAN PATENT APPLICATION**
published in accordance with Art. 153(4) EPC

(43) Date of publication:
**11.06.2025   Bulletin 2025/24**

(21) Application number: 23850152.2

(22) Date of filing: 03.08.2023

(51) International Patent Classification (IPC):
$A61K\ 31/454^{(2006.01)}$   $A61K\ 9/20^{(2006.01)}$
$A61K\ 9/48^{(2006.01)}$   $A61K\ 47/38^{(2006.01)}$
$A61P\ 1/04^{(2006.01)}$   $A61P\ 1/16^{(2006.01)}$
$A61P\ 7/00^{(2006.01)}$   $A61P\ 9/00^{(2006.01)}$
$A61P\ 13/12^{(2006.01)}$   $A61P\ 17/00^{(2006.01)}$
$A61P\ 17/04^{(2006.01)}$   $A61P\ 17/14^{(2006.01)}$
$A61P\ 19/02^{(2006.01)}$   $A61P\ 19/06^{(2006.01)}$
$A61P\ 25/00^{(2006.01)}$   $A61P\ 25/04^{(2006.01)}$
$A61P\ 27/02^{(2006.01)}$   $A61P\ 29/00^{(2006.01)}$
$A61P\ 31/00^{(2006.01)}$   $A61P\ 31/04^{(2006.01)}$
$A61P\ 31/18^{(2006.01)}$

(52) Cooperative Patent Classification (CPC):
**A61K 9/20; A61K 9/48; A61K 31/454; A61K 47/38;
A61P 1/04; A61P 1/16; A61P 7/00; A61P 9/00;
A61P 9/10; A61P 13/12; A61P 17/00; A61P 17/04;
A61P 17/14; A61P 19/02; A61P 19/06;**     (Cont.)

(86) International application number:
**PCT/JP2023/028459**

(87) International publication number:
**WO 2024/029599 (08.02.2024 Gazette 2024/06)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **03.08.2022   JP 2022123712**

(71) Applicant: **Mitsubishi Tanabe Pharma Corporation
Osaka-shi, Osaka 541-8505 (JP)**

(72) Inventors:
• **SUZUKI, Akihiro
  Osaka-shi, Osaka 541-8505 (JP)**
• **TOKUDA, Takayuki
  Osaka-shi, Osaka 541-8505 (JP)**
• **UEHARA, Tomoyuki
  Osaka-shi, Osaka 541-8505 (JP)**

(74) Representative: **J A Kemp LLP
80 Turnmill Street
London EC1M 5QU (GB)**

(54) **PHARMACEUTICAL COMPOSITION CONTAINING 1-{2-[(3S,4R)-1-{[(3R,4R)-1-CYCLOPENTYL-3-FLUORO-4-(4-METHOXYPHENYL)PYRROLIDINE-3-YL]CARBONYL}-4-(METHOXYMETHYL)PYRROLIDINE-3-YL]-5-(TRIFLUOROMETHYL)PHENYL}PIPERIDINE-4-CARBOXYLIC ACID OR PHARMACEUTICALLY ACCEPTABLE SALT OR CO-CRYSTAL THEREOF**

(57)   A pharmaceutical composition containing 1-{2-[(3S,4R)-1-{[(3R,4R)-1-cyclopentyl-3-fluoro-4-(4-methoxyphenyl)pyrrolidin-3-yl]carbonyl}-4-(methoxymethyl)pyrrolidin-3-yl]-5-(trifluoromethyl)phenyl}piperidine-4-carboxylic acid (Compound A) or a pharmaceutically acceptable salt or co-crystal thereof as an active ingredient, wherein a content of Compound A is 30% by weight or more based on a total weight of the pharmaceutical composition. The pharmaceutical composition is preferably obtained using a production method that includes a granulation step of obtaining a granulated product by spraying a binding solution containing Compound A or a pharmaceutically acceptable salt or co-crystal thereof and a binder onto a powder containing Compound A or a pharmaceutically acceptable salt or co-crystal thereof in a fluidized bed granulator.

**(Cont. next page)**

Fig. 1

(52) Cooperative Patent Classification (CPC): (Cont.)
      **A61P 25/00; A61P 25/04; A61P 27/02;**
      **A61P 29/00; A61P 31/00; A61P 31/04;**
      **A61P 31/18; A61P 35/00; A61P 37/06;**
      **A61P 43/00**

## Description

[Technical Field]

The present invention relates to a novel pharmaceutical composition and a method for producing the same, the pharmaceutical composition containing as an active ingredient 1-{2-[(3S,4R)-1-{[(3R,4R)-1-cyclopentyl-3-fluoro-4-(4-methoxyphenyl)pyrrolidin-3-yl]carbonyl} -4-(methoxymethyl)pyrrolidin-3-yl]-5-(trifluoromethyl)phenyl} piperidine-4-carboxylic acid (hereinafter referred to as Compound A) or a pharmaceutically acceptable salt or co-crystal thereof (hereinafter, Compound A or a pharmaceutically acceptable salt or co-crystal thereof will be referred to as Compound A or the like).

Patent Document 1 discloses that multiple pyrrolidine compounds containing Compound A or pharmaceutically acceptable salts thereof, or co-crystals thereof, or the like, have an excellent melanocortin receptor (MCR) activation effect. Further, Patent Document 2 discloses a co-crystal of Compound A.

[Related Art]

[Patent Documents]

[Patent Document 1] WO 2015/182723

[Patent Document 2] WO 2020/138481

[Summary of the Invention]

[Problems to Be Solved by the Invention]

As described in Patent Document 1, Compound A or the like exhibits an excellent MCR activation effect and is therefore expected to be used as a pharmaceutically active ingredient. However, a powder of this compound has chemical properties such as high adhesiveness and water repellency, leaving room for improvement in its formulation.

Specifically, studies of the inventors of the present invention have revealed that when formulating Compound A or the like, problems arise such as difficulties in granulation due to poor powder flowability and adhesion to a container during granulation process, or occurrence of sticking during tableting process. In addition, it was found that solving these problems becomes even more important when a content rate of Compound A or the like in one tablet is increased in order to reduce the size of the tablet.

Therefore, the present invention is intended to provide a novel pharmaceutical composition containing Compound A or the like as an active ingredient, in particular to provide a formulation technology that overcomes problems encountered during production of the pharmaceutical composition, such as troubles encountered during fluidized bed granulation and, when manufacturing tablets, the occurrence of sticking during tableting process, thereby achieving a good yield and/or a reduced rate of defective tablets, and also to provide a pharmaceutical composition with a high content of Compound A or the like that overcomes the problems encountered during production.

[Means for Solving the Problems]

The inventors of the present invention have conducted extensive studies to solve the above-described problems. As a result, it was found that powder flowability can be improved by reducing the amount of Compound A or the like charged as a powder in a container of a fluidized bed granulator. Further, it was found that by spraying a binding solution containing Compound A or the like and a binder onto a powder in a container of a fluidized bed granulator to perform granulation, sticking is reduced when the resulting granulated product is tableted into tablets, and a rate of defective tablets is reduced, and thus the present invention is completed. Further, in particular, when producing a formulation containing Compound A or the like at a high content rate, by spraying a binding solution containing Compound A or the like and a binder onto a powder containing Compound A or the like to perform granulation, not only the two problems described above are solved but all other problems such as adhesion to a container are also solved, and this leads to successful development of a formulation containing Compound A or the like at a high content rate.

A summary of the present invention is as follows.

[1] A pharmaceutical composition containing 1-{2-[(3S,4R)-1-{[(3R,4R)-1-cyclopentyl-3-fluoro-4-(4-methoxyphenyl)pyrrolidin-3-yl]carbonyl}-4-(methoxymethyl)pyrrolidin-3-yl]-5-(trifluoromethyl)phenyl}piperidine-4-carboxylic acid (Compound A) or a pharmaceutically acceptable salt or co-crystal thereof as an active ingredient, wherein a content of Compound A or a pharmaceutically acceptable salt or co-crystal thereof is 30% by weight or more converted to Compound A, based on a total weight of the pharmaceutical composition.

[2] The pharmaceutical composition according to [1], further containing one or more selected from a disintegrant, a binder, and a filler.

[3] The pharmaceutical composition according to [2], wherein a content of the disintegrant is 0.1% - 30% by weight, based on the total weight of the pharmaceutical composition.

[4] The pharmaceutical composition according to [2] or [3], wherein the disintegrant is calcium carmellose or low-substituted hydroxypropyl cellulose.

[5] The pharmaceutical composition according to any one of [2] - [4], wherein a content of the binder is 1% - 15% by weight, based on the total weight of the pharmaceutical composition.

[6] The pharmaceutical composition according to any one of [2] - [5], wherein the binder is a water-soluble polymer.

[7] The pharmaceutical composition according to [6], wherein the water-soluble polymer is hydroxypropyl cellulose.

[8] The pharmaceutical composition according to any one of [2] - [7], wherein a content of the filler is 10% - 50% by weight, based on the total weight of the pharmaceutical composition.

[9] The pharmaceutical composition according to any one of [2] - [8], wherein the filler is lactose hydrate.

[10] The pharmaceutical composition according to any one of [1] - [9], further containing a lubricant.

[11] The pharmaceutical composition according to [10], wherein a content of the lubricant is 0.1% - 5% by weight, based on the total weight of the pharmaceutical composition.

[12] The pharmaceutical composition according to [10] or [11], wherein the lubricant is magnesium stearate.

[13] The pharmaceutical composition according to any one of [1] - [12], wherein the content of Compound A or a pharmaceutically acceptable salt or co-crystal thereof is 50% by weight or more converted to Compound A, based on the total weight of the pharmaceutical composition.

[14] The pharmaceutical composition according to any one of [1] - [13], being a granulated product of Compound A or a pharmaceutically acceptable salt or co-crystal thereof, a capsule formulation containing the granulated product, or a tablet obtained by tableting the granulated product.

[15] The solid pharmaceutical composition according to [14], wherein the granulated product is obtained by granulating a powder containing Compound A or a pharmaceutically acceptable salt or co-crystal thereof by fluidized bed granulation.

[16] The pharmaceutical composition according to any one of [1] - [15], wherein the granulated product is composed of a portion that contains Compound A or a pharmaceutically acceptable salt or co-crystal thereof but substantially does not contain a binder, and a portion that contains Compound A or a pharmaceutically acceptable salt or co-crystal thereof and a binder.

[17] The pharmaceutical composition according to any one of [1] - [16], wherein the granulated product is obtained by spraying a binding solution containing Compound A or a pharmaceutically acceptable salt or co-crystal thereof and a binder onto a powder that may contain Compound A or a pharmaceutically acceptable salt or co-crystal thereof.

[18] The pharmaceutical composition according to any one of [1] - [17], wherein the granulated product is obtained by spraying a binding solution containing Compound A or a pharmaceutically acceptable salt or co-crystal thereof and a binder onto a powder containing Compound A or a pharmaceutically acceptable salt or co-crystal thereof.

[19] The pharmaceutical composition according to [17] or [18], wherein a ratio of a total weight of Compound A or a pharmaceutically acceptable salt or co-crystal thereof in the powder to a total weight of Compound A or a pharmaceutically acceptable salt or co-crystal thereof in the binding solution is 70:30 - 0:100.

[20] The pharmaceutical composition according to any one of [14] - [19], wherein a median particle size of the granulated product is 90 μm - 400 μm.

[21] The pharmaceutical composition according to any one of [14] - [20], wherein an angle of repose of the granulated product is 50 degrees or less.

[22] The pharmaceutical composition according to any one of [14] - [21], being a tablet obtained by tableting the granulated product.

[23] The pharmaceutical composition according to [22], wherein the tablet has a hardness of 30 N or more.

[24] The pharmaceutical composition according to [22] or [23], wherein when 6.5 g of the tablet is tested using a drum described in a friability test method for tablets in Japanese Pharmacopoeia, at a drum rotation speed of 25 rpm for 4 minutes, friability after correction for moisture absorption is 1% or less.

[25] The pharmaceutical composition according to any one of [22] - [24], wherein the tablet shows an individual degradation product amount of 1% or less in a 6-month stability test under conditions of a temperature of 40 °C and a relative humidity of 75%.

[26] The pharmaceutical composition according to any one of [1] - [25], wherein Compound A or a pharmaceutically acceptable salt or co-crystal thereof is a phosphate co-crystal of Compound A.

[27] A method for producing the pharmaceutical composition according to any one of [1] - [26], comprising: a granulation step of obtaining a granulated product by fluidized bed granulation.

[28] A method for producing the pharmaceutical composition according to any one of [1] - [26], comprising: a granulation step of obtaining a granulated product by spraying a binding solution containing Compound A or a pharmaceutically acceptable salt or co-crystal thereof and a binder onto a powder that may contain Compound A or a pharmaceutically acceptable salt or co-crystal thereof in a fluidized bed granulator.

[29] A method for producing a pharmaceutical composition containing 1-{2-[(3S,4R)-1-{[(3R,4R)-1-cyclopentyl-3-fluoro-4-(4-methoxyphenyl)pyrrolidin-3-yl]carbonyl} -4-(methoxymethyl)pyrrolidin-3-yl]-5-(trifluoromethyl)phenyl} piperidine-4-carboxylic acid (Compound A) or a pharmaceutically acceptable salt or co-crystal thereof as an active ingredient, the method comprising: a granulation step of obtaining a granulated product by spraying a binding solution containing Compound A or a pharmaceutically acceptable salt or co-crystal thereof and a binder onto a powder containing Compound A or a pharmaceutically acceptable salt or co-crystal thereof in a fluidized bed granulator.

[30] A method for producing the pharmaceutical composition according to any one of [1] - [26], comprising: a granulation step of obtaining a granulated product by spraying a binding solution containing Compound A or a pharmaceutically acceptable salt or co-crystal thereof and a binder onto a powder containing Compound A or a pharmaceutically acceptable salt or co-crystal thereof in a fluidized bed granulator.

[31] The method according to any one of [27] - [30], comprising: prior to the granulation step, a first mixing step of obtaining a mixture by mixing Compound A or a pharmaceutically acceptable salt or co-crystal thereof with a filler and/or a disintegrant, or a first mixing step of obtaining a mixture by mixing only a filler and a disintegrant.

[32] The method according to [31], comprising: prior to the granulation step, a first mixing step of obtaining a mixture by mixing Compound A or a pharmaceutically acceptable salt or co-crystal thereof with a filler and/or a disintegrant.

[33] The method according to any one of [27] - [32], comprising: a second mixing step of further mixing a disintegrant and/or a lubricant with a granulated product obtained from the granulation step.

[34] The method according to any one of [27] - [33], wherein a ratio of a total weight of Compound A or a pharmaceutically acceptable salt or co-crystal thereof in the powder used in the granulation step to a total weight of Compound A or a pharmaceutically acceptable salt or co-crystal thereof in the binding solution is 70:30 - 0: 100.

[35] The method according to any one of [27] - [34], wherein a ratio of a total weight of Compound A or a pharmaceutically acceptable salt or co-crystal thereof in the powder used in the granulation step to a total weight of Compound A or a pharmaceutically acceptable salt or co-crystal thereof in the binding solution is 65:35 - 0:100.

[36] The method according to any one of [27] - [35], wherein a ratio of a total weight of Compound A or a pharmaceutically acceptable salt or co-crystal thereof in the powder used in the granulation step to a total weight of Compound A or a pharmaceutically acceptable salt or co-crystal thereof in the binding solution is 50:50 - 30:70.

[37] The method according to [36], wherein in the granulation step, a content of Compound A or a pharmaceutically acceptable salt or co-crystal thereof contained in a resulting solid pharmaceutical composition is 50% by weight or more converted to Compound A, based on the total weight of the pharmaceutical composition.

[38] The method according to [37], wherein in the granulation step, a content of Compound A or a pharmaceutically acceptable salt or co-crystal thereof contained in a resulting pharmaceutical composition is 50% by weight converted to Compound A, based on the total weight of the pharmaceutical composition, and a ratio of a total weight of Compound A or a pharmaceutically acceptable salt or co-crystal thereof in the powder to a total weight of Compound A or a pharmaceutically acceptable salt or co-crystal thereof in the binding solution is 50:50.

[39] The method according to [37], wherein in the granulation step, a content of Compound A or a pharmaceutically acceptable salt or co-crystal thereof contained in a resulting pharmaceutical composition is 50% by weight converted to Compound A, based on the total weight of the pharmaceutical composition, and a ratio of a total weight of Compound A or a pharmaceutically acceptable salt or co-crystal thereof in the powder to a total weight of Compound A or a pharmaceutically acceptable salt or co-crystal thereof in the binding solution is 30:70.

[40] The method according to any one of [27] - [39], further comprising: a drying step of drying the granulated product.

[41] The method according to any one of [27] - [40], further comprising: a tableting step of tableting a granulated product obtained from the second mixing step.

[42] The method of [41], further comprising: a coating step of coating a tablet obtained from the tableting step.

[43] A pharmaceutical composition containing 1-{2-[(3S,4R)-1-{[(3R,4R)-1-cyclopentyl-3-fluoro-4-(4-methoxyphenyl)pyrrolidin-3-yl]carbonyl   }-4-(methoxymethyl)pyrrolidin-3-yl]-5-(trifluoromethyl)phenyl}piperidine-4-carboxylic acid (Compound A) or a pharmaceutically acceptable salt or co-crystal thereof as an active ingredient, the pharmaceutical composition being produced using a method comprising: a granulation step of obtaining a granulated product by spraying a binding solution containing Compound A or a pharmaceutically acceptable salt or co-crystal thereof and a binder onto a powder containing Compound A or a pharmaceutically acceptable salt or co-crystal thereof in a fluidized bed granulator.

[Effect of the Invention]

The pharmaceutical composition of the present invention has an advantage that a granulated product containing Compound A or the like, as well as a tablet containing the granulated product, can be obtained efficiently without any trouble during a production step. Further, the pharmaceutical composition of the present invention can contain Compound A or the like at a high ratio and thus has an advantage of allowing a pharmaceutical composition that has an appropriate drug release property and exhibits an expected pharmacological effect to be provided in a more compact form.

[Brief Description of the Drawings]

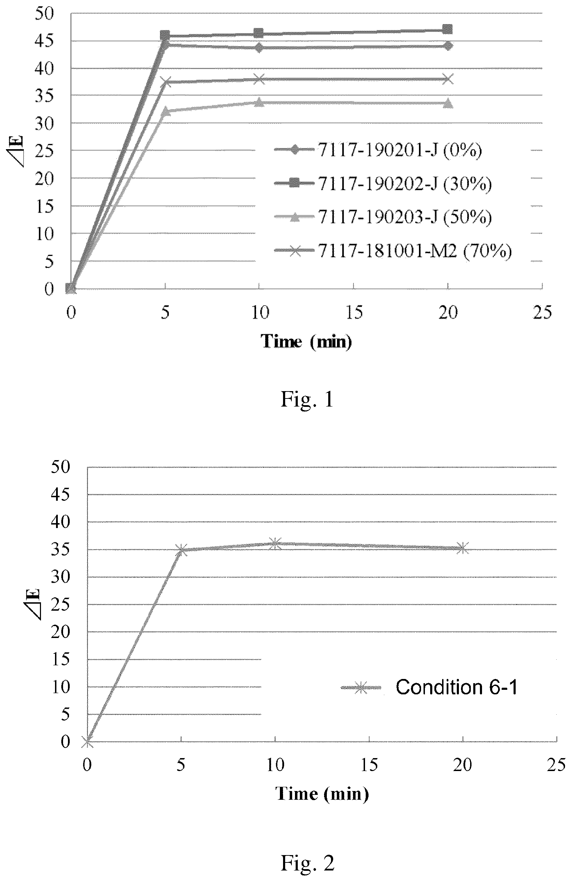

[Fig. 1] A graph showing the change over time in color difference (ΔE) of a punch, measured using a colorimeter, during a step of obtaining a tablet by tableting a granulated product obtained by performing fluidized bed granulation by varying a weight ratio of Compound A or the like charged as powder into a fluidized bed granulator to Compound A or the like added to a binding solution in Example 2. 7117-190201-J (0%) indicates Condition 2-1, 7117-190202-J (30%) indicates Condition 2-2, 7117-190203-J (50%) indicates Condition 2-3, and 7117-181001-M2 (70%) indicates Condition 2-4.

[Fig. 2] A graph showing the change over time in color difference (ΔE) of a punch, measured using a colorimeter, during a step of obtaining a tablet by tableting a granulated product obtained by performing fluidized bed granulation by setting a weight ratio of Compound A or the like charged as powder into a fluidized bed granulator to Compound A or the like added to a binding solution to 7:3 in Example 6. Condition 6-1 is shown.

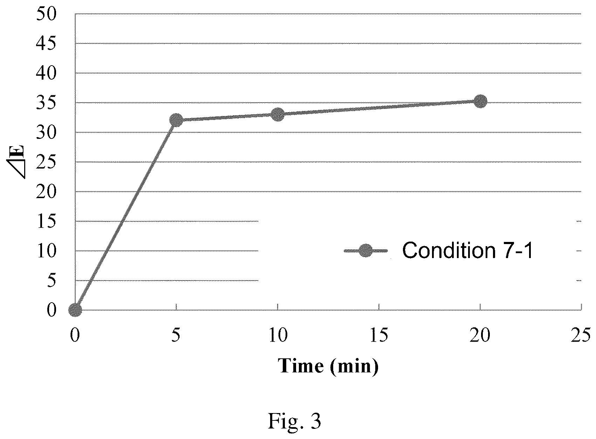

[Fig. 3] A graph showing the change over time in color difference (ΔE) of a punch, measured using a colorimeter, during a step of obtaining a tablet by tableting a granulated product obtained by performing fluidized bed granulation by setting a weight ratio of Compound A or the like charged as powder into a fluidized bed granulator to Compound A or the like added to a binding solution to 3:7 in Example 7. Condition 7-1 is shown.

[Mode for Carrying Out the Invention]

<Pharmaceutical Composition>

As a feature of the present invention, a pharmaceutical composition is characterized in that a content converted to Compound A is 30% by weight or more based on a total weight of the pharmaceutical composition. The content converted to Compound A, based on the total weight of the pharmaceutical composition, is preferably 30% by weight or more and 90% by weight or less, more preferably 30% by weight or more and 80% by weight or less, even more preferably 30% by weight or more and 70% by weight or less, and particularly preferably 30% by weight or more and 60% by weight or less. As another embodiment, the content converted to Compound A is 30% by weight to 40% by weight based on the total weight of the pharmaceutical composition. Further, as another preferred embodiment, the content converted to Compound A, based on the total weight of the pharmaceutical composition, is 40% by weight or more, preferably, 40% by weight or more and 90% by weight or less, more preferably 40% by weight to 80% by weight, even more preferably 40% by weight to 70% by weight, and particularly preferably 40% by weight to 60% by weight. As another preferable example, the content converted to Compound A, based on the total weight of the pharmaceutical composition, is 50% by weight or more, preferably, 50% by weight or more and 90% by weight or less, more preferably 50% by weight to 80% by weight, even more preferably 50% by weight to 70% by weight, and particularly preferably 50% by weight to 60% by weight. Examples of a pharmaceutical composition containing Compound A or the like at a high ratio as an active ingredient include those where the content converted to Compound A is 50% by weight or more based on the total weight of the pharmaceutical composition, and a pharmaceutical composition in which the content is 50% by weight is particularly preferred.

In the present specification, "converted to Compound A" refers to converting to Compound A itself, that is, to a free form of Compound A, for example, converting to an amount of Compound A itself.

Another embodiment of the pharmaceutical composition of the present invention is a pharmaceutical composition containing a granulated product obtained by fluidized bed granulation, in which a weight ratio of Compound A or the like in a powder to Compound A or the like in a binding solution during granulation is preferably between 70:30 and 0:100, and more preferably between 65:35 and 0:100. Particularly preferred examples include those where the weight ratio of Compound A or the like in the powder to Compound A or the like in the binding solution during granulation is between 50:50 and 0:100.

Further, a pharmaceutical composition containing Compound A or the like at a high ratio as an active ingredient is characterized in that a content converted to Compound A is 30% by weight or more based on a total weight of the pharmaceutical composition, and a weight ratio of Compound A or the like in a powder to Compound A or the like in a binding solution during granulation is

(1) between 65:35 and 0:100,

(2) between 60:40 and 0:100,

(3) between 50:50 and 0:100,

(4) between 65:35 and 30:70,

(5) between 60:40 and 30:70, or

(6) between 50:50 and 30:70.

Other preferred examples of the pharmaceutical composition are characterized in that

the content converted to the free form of Compound A is 30% or more and 90% or less by weight based on the total weight of the pharmaceutical composition, and the weight ratio of Compound A or the like in the powder to Compound A or the like in the binding solution during granulation is

(1) between 65:35 and 0:100,

(2) between 60:40 and 0: 100,

(3) between 50:50 and 0:100,

(4) between 65:35 and 30:70,

(5) between 60:40 and 30:70, or

(6) between 50:50 and 30:70; or

the content converted to Compound A is 30% or more and 80% or less by weight based on the total weight of the pharmaceutical composition, and the weight ratio of Compound A or the like in the powder to Compound A or the like in the binding solution during granulation is

(1) between 65:35 and 0:100,

(2) between 60:40 and 0: 100,

(3) between 50:50 and 0:100,

(4) between 65:35 and 30:70,

(5) between 60:40 and 30:70, or

(6) between 50:50 and 30:70; or

the content converted to Compound A is 30% or more and 70% or less by weight based on the total weight of the pharmaceutical composition, and the weight ratio of Compound A or the like in the powder to Compound A or the like in the binding solution during granulation is

(1) between 65:35 and 0:100,

(2) between 60:40 and 0: 100,

(3) between 50:50 and 0:100,

(4) between 65:35 and 30:70,

(5) between 60:40 and 30:70, or

(6) between 50:50 and 30:70; or

the content converted to Compound A is 30% or more and 60% or less by weight based on the total weight of the pharmaceutical composition, and the weight ratio of Compound A or the like in the powder to Compound A or the like in the binding solution during granulation is

(1) between 65:35 and 0:100,

(2) between 60:40 and 0: 100,

(3) between 50:50 and 0:100,

(4) between 65:35 and 30:70,

(5) between 60:40 and 30:70, or

(6) between 50:50 and 30:70, or

the content converted to Compound A is 30% or more and 50% or less by weight based on the total weight of the pharmaceutical composition, and the weight ratio of Compound A or the like in the powder to Compound A or the like in the binding solution during granulation is

(1) between 65:35 and 0: 100,

(2) between 60:40 and 0: 100,

(3) between 50:50 and 0:100,

(4) between 65:35 and 30:70,

(5) between 60:40 and 30:70, or

(6) between 50:50 and 30:70, or

the content converted to Compound A is 30% or more and 40% or less by weight based on the total weight of the pharmaceutical composition, and the weight ratio of Compound A or the like in the powder to Compound A or the like in the binding solution during granulation is

(1) between 70:30 and 0: 100,

(2) between 65:35 and 0:100,

(3) between 60:40 and 0: 100,

(4) between 50:50 and 0:100,

(5) between 70:30 and 30:70,

(6) between 65:35 and 30:70,

(7) between 60:40 and 30:70, or

(8) between 50:50 and 30:70.

Particularly preferred examples include: those where the content converted to Compound A is 30% or more and 60% or less by weight based on the total weight of the pharmaceutical composition, and the weight ratio of Compound A or the like in the powder to Compound A or the like in the binding solution during granulation is between 50:50 and 30:70; or those where the content converted to Compound A is 30% or more and 50% or less by weight based on the total weight of the pharmaceutical composition, and the weight ratio of Compound A or the like in the powder to Compound A or the like in the binding solution during granulation is between 50:50 and 30:70. It is also preferred that the pharmaceutical compositions described above are each in a form of granules containing a granulated product obtained by fluidized bed granulation, or a capsule formulation containing the granulated product, or a tablet obtained by tableting the granulated product.

Other preferred examples of a pharmaceutical composition containing Compound A or the like at a high ratio as an active ingredient include those where the content converted to Compound A is 50% by weight or more based on the total weight of the pharmaceutical composition, and the weight ratio of Compound A or the like in the powder to Compound A or the like in the binding solution during granulation is between 50:50 and 30:70. Particularly preferred examples include those where the content converted to Compound A is 50% or more by weight based on the total weight of the pharmaceutical composition, and the weight ratio of Compound A or the like in the powder to Compound A or the like in the binding solution during granulation is 50:50.

Additionally, particularly preferred examples include those where the content converted to Compound A is 50% or more by weight based on the total weight of the pharmaceutical composition, and the weight ratio of Compound A or the like in the powder to Compound A or the like in the binding solution during granulation is 30:70. It is also preferred that the pharmaceutical compositions described above are each in a form of granules containing a granulated product obtained by fluidized bed granulation, or a capsule formulation containing the granulated product, or a tablet obtained by tableting the granulated product, and the pharmaceutical compositions may be further subjected to a surface coating treatment.

Even more preferred examples of a pharmaceutical composition containing Compound A or the like at a high ratio as an active ingredient include those where the content converted to Compound A is 50% by weight based on the total weight of the pharmaceutical composition, and the weight ratio of Compound A or the like in the powder to Compound A or the like in the binding solution during granulation is between 50:50 and 30:70. Particularly preferred examples include those where the content converted to Compound A is 50% by weight based on the total weight of the pharmaceutical composition, and the weight ratio of Compound A or the like in the powder to Compound A or the like in the binding solution during granulation is 50:50. Additionally, particularly preferred examples include those where the content converted to Compound A is 50% by weight based on the total weight of the pharmaceutical composition, and the weight ratio of Compound A or the like in the powder to Compound A or the like in the binding solution during granulation is 30:70. It is also preferred that the pharmaceutical compositions described above are each in a form of granules containing a granulated product obtained by fluidized bed granulation, or a capsule formulation containing the granulated product, or a tablet obtained by tableting the granulated product, and the pharmaceutical compositions may be further subjected to a surface coating treatment.

Further, examples of a pharmaceutical composition containing Compound A or the like at a high ratio as an active ingredient include those where the content converted to Compound A is 30% by weight or more, preferably 50% by weight or more, based on the total weight of the pharmaceutical composition, and the weight ratio of Compound A or the like in the powder to Compound A or the like in the binding solution during granulation is (less than $(100 - (1.23X - 15.79))$) : ($(1.23X - 15.79)$ or more) when the content of Compound A or the like converted to Compound A is X% by weight based on the total weight of the pharmaceutical composition. It is also preferred that the pharmaceutical compositions described above are each in a form of granules containing a granulated product obtained by fluidized bed granulation, or a capsule formulation containing the granulated product, or a tablet obtained by tableting the granulated product, and the pharmaceutical compositions may be further subjected to a surface coating treatment.

Compound A, that is, 1-{2-[(3S,4R)-1-{[(3R,4R)-1-cyclopentyl-3-fluoro-4-(4-methoxyphenyl)pyrrolidin-3-yl]carbonyl}-4-(methoxymethyl)pyrrolidin-3-yl]-5-(trifluoromethyl)phenyl}piperidine-4-carboxylic acid, or pharmaceutically acceptable salt or co-crystal thereof, which is an active ingredient of the pharmaceutical composition of the present invention, is described in Patent Documents 1 and 2, and can be produced using a method or the like described in these documents.

In the present invention, Compound A can take a form of a pharmaceutically acceptable salt or co-crystal. A preferred embodiment is a co-crystal of Compound A, with a co-crystal with phosphoric acid, especially one equivalent of phosphoric acid, being preferred. A co-crystal of Compound A and phosphoric acid can be obtained using a conventional method, for example, using a method described in Patent Document 2.

In the present invention, a phosphate co-crystal of Compound A can be a compound having a contact angle of 48 to 57 degrees, preferably 50 to 55 degrees. The contact angle is a value obtained using a method described in an example to be described below.

The pharmaceutical composition of the present invention may contain other ingredients in addition to Compound A or a pharmaceutically acceptable salt or co-crystal thereof (which may be referred to as "Compound A or the like" hereinafter).

Examples of the other ingredients include pharmaceutically acceptable additives.

Examples of "pharmaceutically acceptable additives" include fillers, disintegrants, binders, lubricants, and, when necessary, coating agents, coating aids, colorants, masking agents, plasticizers, stabilizers, sweeteners, flavor correctors, antioxidants, glossing agents, flavoring agents, fragrances, defoamers, chewing agents, refreshing agents, sugarcoating agents, foaming agents, disintegration aids, fluidizing agents, and the like, which are commonly used in the art depending on a desired dosage form of the pharmaceutical composition.

As a disintegrant, a commonly used disintegrant added to a pharmaceutical can be used, and there is no particular restriction on its type, and examples thereof include low-substituted hydroxypropyl cellulose, carboxymethyl cellulose, calcium carboxymethyl cellulose, sodium carboxymethyl cellulose, calcium carmellose, sodium carmellose, croscarmellose sodium, crospovidone, starch, crystalline cellulose, hydroxypropyl starch, sodium carboxymethyl starch, partially α-gelatinized starch, and the like. One or more of these can be used. However, calcium carmellose, sodium carboxymethyl starch, partially α-gelatinized starch, low-substituted hydroxypropyl cellulose, and croscarmellose sodium are preferred, with calcium carmellose and low-substituted hydroxypropyl cellulose being more preferable.

Further, water absorption of the disintegrant is preferably 4 mL/g or more, and more preferably 6 mL/g or more. Specifically, calcium carmellose and low-substituted hydroxypropyl cellulose or the like are preferred.

The content of the disintegrant can be appropriately adjusted depending on the dosage form or the like, and is preferably 0.1% to 30% by weight based on the total weight of the pharmaceutical composition. Examples include those where the content is more preferably 1% to 15% by weight, or 3% to 15% by weight, and particularly preferably 5% to 10% by weight.

As a filler, a commonly used filler added to a pharmaceutical can be used, and there is no particular restriction on its type, and examples thereof include mannitol, xylitol, sorbitol, erythritol, maltitol, glucose, white sugar, lactose, crystalline cellulose, calcium hydrogen phosphate, calcium phosphate, wheat starch, rice starch, corn starch, potato starch, sodium carboxymethyl starch, dextrin, $\alpha$-dextrin, $\beta$-dextrin, carboxyvinyl polymer, light anhydrous silicic acid, magnesium oxide, magnesium hydroxide, sodium hydrogen carbonate, magnesium aluminometasilicate, polyethylene glycol, medium chain triglyceride, and the like. One or more of these can be used. However, lactose (hydrated or anhydrous), D-mannitol, crystalline cellulose, anhydrous calcium hydrogen phosphate, and corn starch are preferred, with lactose being more preferred, and lactose hydrate being even more preferred.

The content of the filler can be appropriately adjusted depending on the dosage form, and is preferably 10% to 50% by weight based on the total weight of the pharmaceutical composition. Examples include those where the content is more preferably 20% to 50% by weight, or 20% to 40% by weight, and particularly preferably 20% to 30% by weight.

As a binder, a commonly used binder added to a pharmaceutical can be used, and there is no particular restriction on its type, but a water-soluble polymer is preferably used, and examples thereof include carmellose, carmellose sodium, copolyvidone, pregelatinized starch, hydroxyethyl cellulose, hydroxypropyl cellulose (HPC), hypromellose (hydroxypropyl methylcellulose), pullulan, polyvinylpyrrolidone (povidone), polyvinyl alcohol, gelatin, gum arabic, agar, talagant, sodium alginate, propylene glycol alginate, and the like. One or more of these may be used. However, it is preferable to use HPC, hypromellose, povidone, or polyvinyl alcohol, and it is even more preferable to use HPC.

The content of the binder can be appropriately adjusted depending on the dosage form, and is preferably 1% to 15% by weight based on the total weight of the pharmaceutical composition. Examples include those where the content is more preferably 1% to 10% by weight, or 2% to 10% by weight, and particularly preferably 2% to 5% by weight.

As a lubricant, a commonly used lubricant added to a pharmaceutical can be used, and there is no particular restriction on its type, and examples thereof include stearic acid, magnesium stearate, calcium stearate, polyoxyl stearate, sodium stearyl fumarate, magnesium aluminometasilicate, cetanol, talc, hydrogenated oil, sucrose fatty acid ester, glycerin behenate, light anhydrous silicic acid, hydrated silicon dioxide, dimethylpolysiloxane, microcrystalline wax, beeswax, white beeswax, and the like. One or more of these may be used. However, magnesium stearate, calcium stearate, sodium stearyl fumarate, sucrose fatty acid ester, talc, light anhydrous silicic acid, and hydrated silicon dioxide are preferred, with magnesium stearate being more preferred. The content of the lubricant can be appropriately adjusted depending on the dosage form, and is preferably 0.1% to 5% by weight based on the total weight of the pharmaceutical composition. Examples include those where the content is more preferably 0.5% to 4% by weight, or 1% to 4% by weight, and particularly preferably 1% to 3% by weight.

The dosage form of the pharmaceutical composition of the present invention is preferably a solid formulation, and is not particularly limited as long as it is a solid formulation. Examples thereof include granules, tablets, capsules, powders, fine granules, lozenges, and the like. Granules, capsules, and tablets are preferred, and granules containing a granulated product containing Compound A or the like, capsules containing the granulated product, or tablets obtained by tableting the granulated product are preferred. Among these, tablets are preferred, with tablets obtained by tableting a granulated product obtained by fluidized bed granulation being particularly preferred.

The pharmaceutical composition containing Compound A or the like is more preferably a granulated product consisting of a portion that contains Compound A or the like but substantially does not contain a binder and a portion that contains Compound A or the like and a binder, a capsule formulation containing the granulated product, or a tablet obtained by tableting the granulated product. Further, the pharmaceutical composition containing Compound A or the like is more preferably a pharmaceutical composition that is a granulated product consisting of a portion that contains Compound A or the like but substantially does not contain a binder and a portion that contains Compound A or the like and a binder, a capsule formulation containing the granulated product, or a tablet obtained by tableting the granulated product, and contains Compound A or the like at a high content rate.

Additionally, a pharmaceutical composition is also preferable that contains Compound A or the like and is a granulated product where chemical properties of Compound A or the like such as adhesiveness or water repellency are substantially masked by a binder, a capsule formulation containing the granulated product, or a tablet obtained by tableting the granulated product. Further, the pharmaceutical composition containing Compound A or the like is more preferably a pharmaceutical composition that is a granulated product where chemical properties of Compound A or the like such as adhesiveness or water repellency are substantially masked by a binder, a capsule formulation containing the granulated product, or a tablet obtained by tableting the granulated product, and contains Compound A or the like at a high content rate.

Further, additionally, a pharmaceutical composition is also preferable that contains Compound A or the like and is a granulated product suitable for tableting where Compound A or the like is not exposed as powder on a surface of the granulated product, a capsule formulation containing the granulated product, or a tablet obtained by tableting the granulated product. Further, the pharmaceutical composition containing Compound A or the like is more preferably a pharmaceutical composition that is a granulated product suitable for tableting where Compound A or the like is not exposed as a powder on a surface of the granulated product, a capsule formulation containing the granulated product, or a tablet obtained by tableting the granulated product, and contains Compound A or the like at a high content rate.

When the pharmaceutical composition of the present invention is a pharmaceutical formulation composition containing a granulated product containing Compound A or the like, a shape and properties of the granulated product are not particularly limited. However, it is preferable that the granulated product has the following shape and properties.

Particle sizes of the granulated product containing Compound A or the like are not particularly limited. However, a median particle size ($D_{50}$) is preferably 1000 $\mu$m or less, more preferably in a range of 90 $\mu$m to 500 $\mu$m, and particularly preferably in a range of 90 $\mu$m to 400 $\mu$m. The particle sizes of the granulated product can be measured, for example, using a sieving method described below.

Weigh a tare weight of each of sieves (with mesh openings of 710 $\mu$m, 500 $\mu$m, 355 $\mu$m, 250 $\mu$m, 180 $\mu$m, 150 $\mu$m, 106 $\mu$m, and 75 $\mu$m, and a bottom pan) to the nearest 0.1 g. Stack sieves with larger openings sequentially on top of sieves with smaller openings, and place a precisely weighed 5 g sample on the uppermost sieve and cover it with a lid. Shake the sieves for 5 minutes. Carefully remove each sieve from the stack to avoid any loss of the sample. Weigh each sieve again and measure a weight of the sample on each sieve. Calculate a ratio of the sample on each sieve using the total sample weight and the weight on each sieve.

The granulated product containing Compound A or the like preferably has an angle of repose of 50 degrees or less.

The "angle of repose" is an angle formed between a generatrix of a conical pile and a horizontal plane when drug granules are gently dropped onto a horizontal surface. For example, it can be measured according to the following procedure.

Form an angle of repose on a fixed circular plate with a retaining edge for holding a powder layer. Ensure the circular plate does not vibrate. To carefully form a symmetrical cone, it is recommended to adjust a height of a funnel according to a height of the cone. In this case, since the funnel moves, care must be taken to prevent vibration. To minimize an impact of powder falling on a tip of the cone, maintain a height of a lower end of a funnel leg part at about 2 to 4 cm above a peak of an accumulated pile. An angle of repose ($\alpha$) can be determined using the following formula by measuring a height of the cone:

$$\tan(\alpha) = (\text{height})/(0.5 \times (\text{diameter of the circular plate}))$$

The granulated product containing Compound A or the like preferably has a flowability with a minimum orifice diameter of 3.15 to 8 mm, and more preferably has a flowability with a minimum orifice diameter of 3.15 to 4 mm.

A bulk density is a ratio of the mass of a powder sample in an untapped (loose) state to the volume of the powder including a factor of an inter-particle void volume.

Further, a tapped density is an increased bulk density obtained after mechanically tapping a container containing the powder sample.

The granulated product containing Compound A or the like preferably has a Hausner ratio of 1.00 to 1.45. The Hausner ratio is a measure of the compressibility and flowability of a powder. When the Hausner ratio is 1.00 to 1.11, 1.12 to 1.18, 1.19 to 1.25, 1.26 to 1.34, 1.35 to 1.45, 1.46 to 1.59, or 1.60 or more, a degree of flowability is extremely good, good, fairly good, passable, poor, very poor, or extremely poor, respectively.

The Hausner ratio can be calculated by measuring the bulk density ($V_0$) or tapped density ($V_f$) of the granulated product containing Compound A or the like, and using the formula Hausner ratio = $V_0/V_f$.

The pharmaceutical composition of the present invention can also be a pharmaceutical composition containing coated granules obtained by coating the granulated product with a coating agent. As a coating agent used for coating the granulated product, a coating agent commonly used in pharmaceuticals can be used, and its type is not particularly limited. For example, one or more of a water-soluble cellulose derivative, a water-soluble vinyl derivative, a water-soluble acrylic acid derivative, a water-insoluble cellulose derivative, a water-insoluble vinyl derivative, a water-insoluble acrylic acid polymer, a gastric soluble vinyl derivative, a gastric soluble acrylic acid polymer, an enteric cellulose derivative, an enteric vinyl derivative, an enteric acrylic acid polymer, corn protein, shellac, wax, and the like can be used.

The pharmaceutical composition of the present invention may be a capsule formulation obtained by enclosing the granulated product in a capsule.

As the capsule, a material commonly used for pharmaceutical encapsulation can be used. For example, a capsule formed of gelatin, hypromellose, pullulan, or the like can be used. The amount of Compound A or the like in one capsule formulation can be appropriately adjusted depending on a type of disease or other factors, and for example, can be set to 10 mg to 500 mg converted to Compound A.

The pharmaceutical composition of the present invention may be a tablet obtained by tableting the granulated product.

Tableting can be performed using a commonly known method. The amount of Compound A or the like in one tablet can be appropriately adjusted depending on a type of disease or other factors, and for example, can be in a range of 0.1 to 1,000 mg, preferably, in a range of 0.1 to 500 mg, converted to Compound A. More preferably, the amount is in a range of 50 mg to 300 mg, and particularly preferably 50 mg, 100 mg, 200 mg or 300 mg.

Further, when the pharmaceutical composition of the present invention is a tablet, the tablet is preferably excellent in stability and solubility and has one or more of the following properties.

(1) The tablet has a hardness of 30 N or more.

(2) When 6.5 g of the tablet is tested using a drum described in a friability test method for tablets in Japanese Pharmacopoeia, at a drum rotation speed of 25 rpm for 4 minutes, friability after correction for moisture absorption is 1% or less.

(3) An individual degradation product amount in a 6-month stability test under conditions of a temperature of 40 °C and a relative humidity of 75% is 1% or less.

The obtained tablet may be subjected to a surface coating treatment.

As the coating agent, a coating agent commonly used in pharmaceuticals can be used, and it can be appropriately selected according to an intended purpose, such as fast release, sustained release, enteric release, or gastric release. For example, one or more selected from a water-soluble cellulose derivative, a water-soluble vinyl derivative, a water-soluble acrylic acid derivative, a water-insoluble cellulose derivative, a water-insoluble vinyl derivative, a water-insoluble acrylic acid polymer, a gastric soluble vinyl derivative, a gastric soluble acrylic acid polymer, an enteric cellulose derivative, an enteric vinyl derivative, an enteric acrylic acid polymer, corn protein, shellac, wax, and the like can be used.

In addition to a coating agent, various additives may be blended into a coating liquid. Examples of such additives include coating aids, colorants, masking agents, plasticizers, lubricants, and the like. Examples of coating aids include hydrogenated oil, stearic acid and its salts, glycerin monostearate, talc, kaolin, sucrose fatty acid ester, higher alcohols such as cetanol, and the like. Examples of colorants include food dyes, lake pigments, and all other colored substances that can be used in pharmaceuticals. Examples of masking agents include titanium dioxide, precipitated calcium carbonate, dibasic calcium phosphate, calcium sulfate, and the like. Examples of plasticizers include phthalic acid derivatives such as diethyl phthalate, as well as polyethylene glycol, propylene glycol, triacetin, triethyl citrate, glycerin, glycerin fatty acid esters, silicone oil, and the like.

Specific examples of coating liquid include those prepared by combining a base material such as hydroxypropylmethylcellulose, hydroxypropylcellulose, methylcellulose, aminoalkyl methacrylate copolymer E, polyvinyl acetal diethylaminoacetate, polyvinylpyrrolidone, pullulan, or the like with a plasticizer such as polyethylene glycol, propylene glycol, triacetin, triethyl citrate, glycerin, glycerin fatty acid ester, or the like, and, when necessary, adding an additive such as titanium dioxide, talc, iron oxide, precipitated calcium carbonate, dibasic calcium phosphate, calcium sulfate, or the like.

The use of the pharmaceutical composition of the present invention is not particularly limited as long as it is for a disease for which Compound A or the like can exert a therapeutic or preventive effect as an active ingredient. However, examples thereof include diseases that can be treated or prevented via MCR, particularly MC1R activation, as described in Patent Document 1, specifically, autoimmune diseases, inflammatory diseases, and fibrosis diseases.

Examples of such diseases include rheumatoid arthritis, gouty arthritis, osteoarthritis, inflammatory bowel disease, systemic scleroderma, psoriasis, fibrosis, protoporphyria such as erythropoietic protoporphyria), systemic lupus erythematosus, melanoma, skin cancer, vitiligo, alopecia, pain, ischemia/reperfusion injury, brain inflammatory diseases, hepatitis, sepsis/septic shock, nephritis, transplantation, HIV disease exacerbation, vasculitis, uveitis, retinitis pigmentosa, age-related macular degeneration, microbial infections, celiac disease, nephrotic syndrome, melanoma invasion, and the like.

Further, the pharmaceutical composition of the present invention is also useful for treating or preventing photodermatoses (also referred to as photosensitivity), hypopigmentary disorders, side effects of phototherapy, and the like.

Examples of photodermatoses include photoallergic dermatitis, vacciniforme bullous dermatitis, DNA repair disorders, and porphyria.

Examples of photoallergic dermatitis include solar urticaria, polymorphous light eruption, chronic actinic dermatitis, photoallergic contact dermatitis, photoallergic photosensitivity-type drug eruptions, and the like.

Examples of DNA repair disorders include xeroderma pigmentosum, Bloom syndrome, Werner syndrome, Rothmund-Thomson syndrome, trichothiodystrophy, ultraviolet hypersensitivity syndrome, and the like.

Examples of porphyria include congenital erythropoietic porphyria, variegate porphyria, acute intermittent porphyria, porphyria cutanea tarda, hereditary coproporphyria, and the like.

Examples of hypopigmentary disorders include congenital hypopigmentation disorders such as oculocutaneous albinism, piebaldism, hypopigmented nevus, hypopigmentation (Ito's hypomelanosis), phenylketonuria, tuberous sclerosis, hereditary contralateral pigmentary anomaly, Waardenburg syndrome, Hermansky-Pudlak syndrome, Chediak-Higashi syndrome, Griscelli syndrome, and Tietz, as well as acquired hypopigmentation disorders such as Sutton's nevus (halo nevus), Vogt-Koyanagi-Harada disease, senile leukoderma, post-beach bathing hypopigmentation, and syphilitic leukoderma syndrome.

On the other hand, phototherapy is used for treating inflammatory skin diseases, vitiligo, alopecia areata, and the like. Known methods include narrowband UVB therapy, excimer light therapy, PUVA therapy, and the like. However, as side effects, short-term effects include photosensitivity symptoms or phototoxic reactions such as redness, sunburn, and heat sensation in irradiated areas, while long-term side effects include cancer such as skin cancer, and photoaging such as development of spots and wrinkles. Compound A or the like can also protect the skin and exhibit a preventive or therapeutic effect against such side effects of phototherapy.

Further, the pharmaceutical composition of the present invention is also useful for treating or preventing interstitial lung diseases, as well as diseases or symptoms associated with scleroderma. In particular, it is useful for treating or preventing interstitial lung diseases, fibrosis of skin and visceral tissues, vascular dysfunction, and the like in scleroderma patients.

Examples of interstitial lung diseases include interstitial pneumonia. Examples of interstitial pneumonia include idiopathic interstitial pneumonia, particularly, idiopathic interstitial pneumonia accompanied by idiopathic pulmonary fibrosis. Further, examples of interstitial lung diseases include interstitial lung diseases associated with collagen diseases and interstitial lung diseases associated with scleroderma. Here, scleroderma is preferably systemic scleroderma, which includes limited cutaneous systemic sclerosis and diffuse cutaneous systemic sclerosis.

Diseases and symptoms associated with scleroderma include those selected from skin fibrosis, flexion contracture, gastroesophageal reflux disease, dysphagia, Raynaud's phenomenon, digital ulcer, pulmonary hypertension, and renal crisis.

Here, flexion contracture is a symptom caused by skin fibrosis in scleroderma and can be improved by inhibiting skin fibrosis. Gastroesophageal reflux disease and dysphagia are symptoms caused by esophageal fibrosis in scleroderma and can be improved by inhibiting esophageal fibrosis. Raynaud's phenomenon, digital ulcers, pulmonary hypertension, and renal crisis are symptoms caused by vascular dysfunction in scleroderma and can be improved by inhibiting vascular dysfunction. Pulmonary hypertension is not particularly limited, and an example thereof is pulmonary arterial hypertension.

The pharmaceutical composition obtained in the present invention is particularly useful as an oral formulation. The dose varies depending on the patient's symptoms, age, sex, and a desired duration of administration. However, specifically, the dose per administration can be in a range of 0.1 to 1000 mg, preferably in a range of 0.1 to 500 mg, converted to Compound A. More preferably, the does is in a range of 10 to 300 mg, or 100 to 300 mg. For example, specifically, the dose is preferably 50 mg, 100 mg, 200 mg, 300 mg, or the like.

<Production method>

In the following, as one feature of the present invention, a method for producing a pharmaceutical composition containing Compound A or the like, preferably the pharmaceutical composition of the present invention, is described. However, the pharmaceutical composition of the present invention is not limited to those produced using the following method.

The production method of the present invention includes the following granulation step.

A granulation step in which a granulated product is obtained by spraying a binding solution that contains Compound A or a pharmaceutically acceptable salt or co-crystal thereof (Compound A or the like) and a binder onto a powder that substantially does not contain a binder in a fluidized bed granulator.

Compound A or the like may be contained in the powder that substantially does not contain a binder.

One embodiment of the production method of the present invention includes the following granulation step.

A granulation step of obtaining a granulated product by spraying a binding solution containing Compound A or a pharmaceutically acceptable salt or co-crystal thereof (Compound A or the like) and a binder onto a powder that contains Compound A or the like but substantially does not contain a binder in a fluidized bed granulator.

The production method of the present invention according to this embodiment is characterized in that Compound A or the like is contained in both the powder and the binding solution used for the fluidized bed granulation. A ratio of (1) a total weight of Compound A or the like in the powder to (2) a total weight of Compound A or the like in the binding solution is preferably between 70:30 and 0: 100, more preferably between 65:35 and 0: 100, and particularly preferably between 50:50 and 30:70. A preferred range of this weight ratio can be any of the ranges described in the section on the pharmaceutical composition of the present invention described above.

By adjusting the ratio in the above range, it is possible to avoid troubles that occur during granulation and tableting, and to obtain a granulated product suitable for a pharmaceutical composition containing Compound A or the like at a high content rate.

To avoid troubles that occur during granulation, it is preferable to mix Compound A or the like with an excipient and/or a disintegrant, or to mix only a filler and a disintegrant, and use the resulting mixture as a powder for granulation, or to use a filler or a disintegrant alone as a powder. In particular, it is preferable to mix Compound A or the like with a filler and/or a disintegrant and use the resulting mixture as a powder for granulation.

Therefore, the production method of the present invention preferably includes the following mixing step (first mixing step) prior to the granulation step.

A mixing step of obtaining a mixture by mixing Compound A or the like with a filler and/or a disintegrant.

Other ingredients may be further mixed in addition to the filler and/or disintegrant. A more preferred example is one in which the powder for granulation contains Compound A or the like and a filler and/or a disintegrant, but substantially does not contain a binder.

Further, the production method of the present invention preferably includes a drying step of drying the granulated product after the granulation step.

The obtained granulated product can be used as is or coated for use as granules.

Further, the obtained granulated product can be used as is or coated and encapsulated for use as a capsule formulation.

On the other hand, the production method of the present invention may include a tableting step of tableting the obtained granulated product to obtain a tablet.

Here, when a disintegrant and a lubricant are mixed for tableting, a mixing step (second mixing step) of further mixing the disintegrant and the lubricant with the granulated product may be included. In this way, granules obtained, for example, by adding additives or the like necessary for tableting with the granulated product obtained from the granulation step and mixing the resulting mixture may be referred to as "mixed granules" in the present specification. In this specification, since the mixed granules differ only slightly from the granulated product in terms of formulation ingredients, the preferred ranges for the indicators such as the median particle size, the angle of repose, the minimum orifice diameter, the Hausner ratio, and the like of the granulated product also apply to the mixed granules.

The production method of the present invention may further include a step of coating the tablet obtained from the tableting step.

In the following, a production method is described in detail for a case where drug granules are produced by spraying a binding solution containing Compound A or the like onto a powder that may contain Compound A or the like, the resulting granulated product is tableted into a tablet, and the tablet is further coated.

<First mixing step>

First, a powder for granulation is prepared by mixing Compound A or the like with a filler and/or a disintegrant, or by mixing only a filler and a disintegrant, or a powder for granulation is prepared using a filler or a disintegrant alone. Preferably, a powder for granulation is prepared by mixing Compound A or the like with a filler and/or a disintegrant. Other ingredients may be mixed in addition to the filler and/or disintegrant. However, a binder is substantially not contained as another ingredient in addition to a filler and/or a disintegrant.

Compound A or the like used in the granulation is not particularly limited in shape and can be used in various shapes. However, crystals are preferred. The crystals include co-crystals. The crystals may be single crystals or polycrystals. Sizes of a powder of Compound A or the like to be used are not particularly limited as long as the sizes are suitable for granulation. However, a median particle size measured using a method such as direct observation under a microscope or by using an electrical or optical particle size measurement device is preferably 0.1 µm to 100 µm, and more preferably 1 µm to 10 µm.

A filler and a disintegrant to be used are not particularly limited in type, and the fillers and/or disintegrants described above can be selected and used as appropriate. In the first mixing step, when Compound A or a pharmaceutically acceptable salt or co-crystal thereof is mixed with a filler, Compound A or a pharmaceutically acceptable salt or co-crystal thereof is mixed with the filler at a weight ratio of 0:100 to 50:50. More preferably, in the first mixing step, Compound A or a pharmaceutically acceptable salt or co-crystal thereof is mixed with the filler at a weight ratio of 25:75 to 50:50. Further, in the first mixing step, when Compound A or a pharmaceutically acceptable salt or co-crystal thereof is mixed with a disintegrant, Compound A or a pharmaceutically acceptable salt or co-crystal thereof is mixed with the disintegrant at a weight ratio of 0:100 to 50:50. More preferably, in the first mixing step, Compound A or a pharmaceutically acceptable salt or co-crystal thereof is mixed with the disintegrant at a weight ratio of 25:75 to 50:50. In the first mixing step, when Compound A or a pharmaceutically acceptable salt or co-crystal thereof is mixed with a filler and a disintegrant, a ratio of a weight of Compound A or a pharmaceutically acceptable salt or co-crystal thereof to a total weight of the filler and the disintegrant is 0:100 to 50:50. More preferably, the ratio of the weight of Compound A or a pharmaceutically acceptable salt or co-crystal thereof to the total weight of the filler and the disintegrant is 25:75 to 50:50. It is more preferable to use both a filler and a disintegrant. Particularly preferably, Compound A or a pharmaceutically acceptable salt or co-crystal thereof is mixed with a filler and a disintegrant at weight ratios of 47:45:8 or 35:55:10.

As a mixer, for example, a container rotating-type mixer can be used. Examples of container rotating-type mixers include a tumble blender, a V blender, a double cone, a bin tumble, and the like.

<Granulation Step>

In the granulation step, first, a binding solution for granulation is prepared.

Examples of solvents for the binding solution include water, alcohol solvents such as methanol, ethanol, and 2-propanol, and mixtures of these solvents. Among these solvents, water, particularly purified water, is preferred.

By dissolving a binder in such a solvent to prepare a solution and then dispersing Compound A or the like in the resulting solution, a binding solution containing Compound A or the like can be obtained.

As the binder, a substance such as a water-soluble polymer described above can be appropriately selected and used. A concentration of the binder in the binding solution is preferably, for example, 1 to 10% by weight.

A concentration of Compound A or the like in the binding solution (spray solution) is not particularly limited, but is preferably 5% by weight or more converted to a weight of Compound A or the like, more preferably 10% by weight to 60% by weight, and particularly preferable 30% by weight to 50% by weight. Compound A or the like to be dispersed is preferably finely pulverized by dry or wet grinding in order to uniformly disperse a drug in a formulation.

In the granulation step, a total weight of the binding solution to be sprayed is preferably equal to or greater than a total amount of the powder to be used for granulation, more preferably 1 to 3 times the total amount of the powder, and even more preferably 1 to 2 times the total amount of the powder. A weight ratio of an amount of Compound A or the like in the powder to be used for granulation to an amount of Compound A or the like in the binding solution to be sprayed is preferably adjusted to be between 70:30 and 0: 100, and more preferably between 65:35 and 0: 100. In particular, in a pharmaceutical composition containing Compound A or the like at a high content rate, the weight ratio of the amount of Compound A or the like in the powder to be used for granulation to the amount of Compound A or the like in the binding solution to be sprayed is preferably adjusted to be between 65:35 and 30:70, and more preferably between 50:50 and 30:70. Further, a pharmaceutical composition produced using such a method is also preferred.

Granulation can be performed according to a general procedure for fluidized bed granulation.

A granulator to be used is not particularly limited as long as it is a fluidized bed granulator. A spray method may use any of a top spray type, a bottom spray type, and a tangential spray type.

A fluidized bed granulator typically consists of a fluidized bed body, a rectifying plate, a blower, an intake filter, a heat exchanger, a spray device, a dust collector, an exhaust fan, and the like. Air supplied from the blower fan is purified by the intake filter, heated by the heat exchanger, and then sent to a main body of the apparatus through the rectifying plate. This hot air keeps a mixture of Compound A or the like, a filler, and/or a disintegrant loaded in the apparatus in a fluidized state. By spraying a binder solution in which Compound A or the like is suspended as the binding solution, the mist adheres to the surface of the powder. Through this binder mist, the powder fine particles repeatedly adhere and aggregate, gradually progressing particle growth (granulation and coating).

The inlet air temperature during granulation is selected, for example, from a range of 50 °C to 90 °C, and preferably from a range of 60 °C to 80 °C. The liquid feed rate during granulation depends on the scale of the granulator to be used, the charged amount of the mixture of Compound A, the filler, and/or the disintegrant, and desired particle sizes of the granulated product. For example, in the case of a 10 kg charge, the amount of solution is selected from a range of 100 g/min to 300 g/min. The air flow rate during granulation depends on the scale of the granulator to be used, the charged amount of the mixture of Compound A, the filler, and/or the disintegrant, and desired particle sizes of the granulated product. For example, in the case of a 10 kg charge, it is selected from a range of 1 $m^3$/min to 10 $m^3$/min. The spray air volume during granulation depends on the scale of the granulator to be used, the liquid feed rate, and the position of the spray nozzle, and, for example, for a 10 kg charge, it is selected from a range of 100 L/min to 500 L/min. The spray nozzle diameter during granulation depends on the scale of the granulator to be used. For example, in the case of a 10 kg charge with a liquid feed rate of 200 g/min, it is selected from a range of 0.5 mm to 2.0 mm.

<First drying step>

Next, the granulated product obtained from the granulation step is dried. The drying can be performed under a reduced pressure or an atmospheric pressure such that the weight loss due to drying, as measured by an infrared moisture meter, becomes, for example, 3% by weight or less. The drying can be performed in the granulation apparatus. However, it is also possible that the drying is performed after the granules are removed from the granulator.

<Second mixing step>

Next, in preparation for tableting, a disintegrant, a lubricant, and the like are mixed with the granulated product obtained from the drying step. As a mixer, for example, a container rotating-type mixer can be used. Examples of container rotating-type mixers include a tumble blender, a V blender, a double cone, a bin tumble, and the like.

A mixing ratio of the disintegrant can be set, for example, in a range of 0.1% by weight to 10% by weight based on a total weight of the granulated product. A mixing ratio of the lubricant can be set, for example, in a range of 0.1% by weight to 10% by weight based on the total weight of the granulated product.

<Tableting step>

In the present step, the mixture obtained from the second mixing step is tableted into a tablet using a tableting machine. Tablet hardness can be selected, for example, from a range of 20 to 100 N. As a tableting machine, a tableting machine classified as a rotary tableting machine can be used. The tablet obtained here is referred to as an core tablet in the present specification, and when mentioning "the total weight of the pharmaceutical composition" or the like, it refers to the weight of the core tablet. That is, the weight ratio of Compound A or the like in one tablet is based on the weight of the core tablet.

<Coating step>

In the present step, a tablet obtained from the tableting step is coated as appropriate according to an intended purpose. As a coating machine, for example, a machine classified as a coating pan can be used, for example, a machine classified as a perforated coating system can be used.

As a coating agent, the coating agents described above can be used, and can be appropriately selected and used according to an intended purpose, such as fast release, sustained release, enteric release, or gastric release.

An amount of the coating agent relative to the tablet only needs to be sufficient to coat the surface of the tablet, and can be adjusted according to a desired release property.

<Second drying step>

The drying can be performed under a reduced pressure or an atmospheric pressure such that the weight loss due to drying, as measured by an infrared moisture meter, becomes, for example, 3% by weight or less. The drying may be performed in the coating machine or may be performed separately after removing the coated tablet from the coating machine.

[Examples]

In the following, the present invention is specifically described with reference to examples. However, the present invention is not limited to the following examples.

In the following, various test items for the examples and comparative examples were measured as follows.

<Particle size distribution>

Nine types of sieves to be used: sieve openings of 710 $\mu$m, 500 $\mu$m, 355 $\mu$m, 250 $\mu$m, 180 $\mu$m, 150 $\mu$m, 106 $\mu$m, and 75 $\mu$m, and a bottom pan.

Method:

The tare weight of each sieve was measured to the nearest 0.1 g, and sieves with larger mesh openings were sequentially stacked on top of sieves with smaller mesh openings. A precisely weighed 5 g sample was placed on the uppermost sieve and it was covered with a lid, and the sieves were shaken for 5 minutes. Each sieve was carefully removed from the stack of sieves to avoid any sample loss, and the weight of each sieve was reweighed to measure a weight of the sample on each sieve. A ratio of the sample on each sieve was calculated using the total sample weight and the weight on each sieve.

<Bulk density>

A sample was allowed to freely flow into a 100 mL stainless steel cylindrical container using a funnel until it overflowed. After that, excess powder was carefully scraped off from the top of the container. The weight ($m_0$) of the powder was measured by subtracting a pre-measured weight of an empty measuring container. The bulk density (g/mL) was calculated using the formula $m_0/100$.

<Tapped density>

An auxiliary cylinder was attached to the 100 mL stainless steel cylindrical container used for measuring the bulk density, and a sample was allowed to freely flow into the container using a funnel until it overflowed. After that, the measuring container with the auxiliary cylinder was tapped a total of 200 times at a rate of 50 to 60 taps per minute. After the tapping operation, the auxiliary cylinder was removed, and the excess powder was carefully scraped off from the top of the measuring container. The tapped density (g/mL) was calculated using the formula $m_f/100$ (where $m_f$ is the weight of the powder in the measuring container after tapping and scraping).

<Hausner ratio>

The Hausner ratio was calculated using the following formula. Hausner ratio = $\rho_t/\rho_0$ ($\rho_t$: tapped density, $\rho_0$: bulk density)

<Minimum orifice diameter>

Measurable orifice diameters: 3.15 mm, 4 mm, 5 mm, 6.3 mm, 8 mm, 10 mm, 12.5 mm, 16 mm, 20 mm, and 25 mm.
Using a funnel, the sample was gently introduced into a cylindrical container for orifice diameter measurement, and the smallest orifice diameter through which the sample freely fell was measured as the minimum orifice diameter.

[Example 1]

A tablet was obtained by performing fluidized bed granulation using a phosphate co-crystal of Compound A under the following conditions.
Condition 1-1: The weight ratio of the phosphate co-crystal of Compound A charged as powder into the fluidized bed granulator to the phosphate co-crystal of Compound A added to the binding solution was 3:7.
Condition 1-2: The weight ratio of the phosphate co-crystal of Compound A charged as powder into the fluidized bed granulator to the phosphate co-crystal of Compound A added to the binding solution was 5:5.
A preparation step of the binding solution was carried out according to the following procedure.

[1] 0.456 kg of hydroxypropyl cellulose was gradually added to 7.164 kg of purified water, and the mixture was stirred for at least 30 minutes to dissolve.

[2] An arbitrary proportion of 8.702 kg of phosphate co-crystal of Compound A was gradually added to the aqueous solution of [1] while stirring, and the mixture was stirred and dispersed for at least 20 minutes.

[3] The entire suspension prepared in [2] was sieved through a sieve, and it was visually confirmed that there were no undissolved residues of hydroxypropyl cellulose or agglomerates of phosphate co-crystal of Compound A.

[4] 1.5 kg of purified water was added to the SUS container used in [3], any adhering drug substance was washed in, and the mixture was sieved through a sieve to obtain a binding solution.

Granulation and drying were carried out according to the following procedure.

[1] 4.218 kg of lactose hydrate, 0.760 kg of carmellose calcium, and the remaining phosphate co-crystal of Compound A were charged in this order into a fluidized bed granulation dryer.

[2] After preheating and mixing until the exhaust temperature reached 40 °C with the inlet air temperature set to 70 °C, the binding solution spray rate was adjusted to reach a target solution rate, and granulation was performed.

[3] After spraying the entire amount of the binding solution, drying was performed at an intake temperature of 80 °C until the product temperature reached 45 °C, and a granulated product was obtained.

Mixing and tableting were carried out according to the following procedure.

[1] 0.760 kg of calcium carmellose, 0.304 kg of magnesium stearate, and the granulated product were charged into a mixer and mixed at a mixer rotation speed of 25 min$^{-1}$ to obtain mixed granules. The amounts of carmellose calcium and magnesium stearate used in the present step were adjusted according to an actual yield of granulated product, with the above values serving as theoretical values.

[2] The mixed granules from [1] were tableted to obtain an core tablet. A weight ratio of the phosphate co-crystal of Compound A in the core tablet is 50% converted to Compound A.

A coating liquid step and coating step were carried out according to the following procedure.

[1] A coating ingredient was gradually added to purified water while stirring such that a solid content concentration reaches 10 w/w%, and the mixture was stirred for at least 45 minutes.

[2] The mixture was sieved through a sieve to obtain a coating liquid.

[3] The tablet was coated using an aeration coating system.

The results of the production are shown in Table 1.

[Table 1]

| | | Condition 1-1 | Condition 1-2 |
|---|---|---|---|
| Weight ratio of Compound A phosphate co-crystal in core tablet (%) | | 57.25 | 57.25 |
| (1) : (2)<br>(1) Total weight of Compound A phosphate co-crystal in powder<br>(2) Total weight of Compound A phosphate co-crystal in binding solution | | 3:7 | 5:5 |
| Median particle size of granulated product ($\mu$m) | | 138.0 | 127.6 |
| Particle size distribution of granulated product (%) | 710 $\mu$m on | 0.0 | 0.2 |
| | 500 $\mu$m on | 0.1 | 1.4 |
| | 355 $\mu$m on | 0.4 | 1.4 |
| | 250 $\mu$m on | 4.8 | 2.4 |
| | 180 $\mu$m on | 22.3 | 11.6 |
| | 150 $\mu$m on | 14.6 | 18.0 |
| | 106 $\mu$m on | 29.7 | 29.5 |
| | 75 $\mu$m on | 14.8 | 19.6 |
| | 75 $\mu$m pass | 13.3 | 15.8 |
| Minimum orifice diameter of mixed granules (mm) | | 3.15 | 3.15 |
| Bulk density of mixed granules (g/mL) | | 0.38 | 0.48 |
| Tapped density of mixed granules (g/mL) | | 0.50 | 0.63 |
| Hausner ratio of mixed granules | | 1.315 | 1.312 |
| Tableting property (stickiness) | | None | None |
| Core tablet hardness (N) | | 104 | 69 |
| Coated tablet hardness (N) | | 114 | 98 |

In both Condition 1-1 and 1-2, the flowability during granulation was good, no adhesion to the container was observed, and no sticking during tableting was observed.

[Example 2]

A tablet was obtained by performing fluidized bed granulation using a phosphate co-crystal of Compound A under the following conditions.

Condition 2-1: The weight ratio of the phosphate co-crystal of Compound A charged as powder into the fluidized bed granulator to the phosphate co-crystal of Compound A added to the binding solution was 10:0.

Condition 2-2: The weight ratio of the phosphate co-crystal of Compound A charged as powder into the fluidized bed granulator to the phosphate co-crystal of Compound A added to the binding solution was 7:3.

Condition 2-3: The weight ratio of the phosphate co-crystal of Compound A charged as powder into the fluidized bed granulator to the phosphate co-crystal of Compound A added to the binding solution was 5:5.

Condition 2-4: The weight ratio of the phosphate co-crystal of Compound A charged as powder into the fluidized bed granulator to the phosphate co-crystal of Compound A added to the binding solution was 3:7.

A preparation step of the binding solution was carried out according to the following procedure.

[1] 30 g of hydroxypropyl cellulose was gradually added to 470 g of purified water and stirred to dissolve.

[2] The entire suspension prepared in [1] was sieved through a sieve, and it was visually confirmed that there were no undissolved residues of hydroxypropyl cellulose, and a binding solution was obtained.

[3] When necessary, an arbitrary proportion of 572.5 g of phosphate co-crystal of Compound A was gradually added to the aqueous solution of [2] while stirring, and the mixture was stirred and dispersed for at least 20 minutes.

[4] The entire suspension prepared in [3] was sieved through a sieve, and it was visually confirmed that there were no undissolved residues of hydroxypropyl cellulose or agglomerates of phosphate co-crystal of Compound A added when necessary.

[5] 100 g of purified water was added to the SUS container used in [3], any adhering drug substance was washed in, and the mixture was sieved through a sieve to obtain a binding solution.

Granulation and drying were carried out according to the following procedure.

[1] 277.5 g of lactose hydrate, 50 g of carmellose calcium, and the remaining phosphate co-crystal of Compound A were charged in this order into a fluidized bed granulation dryer.

[2] After preheating and mixing with an inlet air temperature set to 70 °C, the binding solution spray rate was adjusted to the target liquid rate and granulation was performed.

[3] After spraying the entire amount of the binding solution, drying was performed at an inlet air temperature of 80 °C until the product temperature reached about 35 °C or higher, and a granulated product was obtained.

Mixing and tableting were carried out according to the following procedure.

[1] 50 g of carmellose calcium, 20 g of magnesium stearate, and the granulated product were mixed 100 times by bag mixing to obtain mixed granules. The amounts of carmellose calcium and magnesium stearate used in the present step were adjusted according to an actual yield of granulated product, with the above values serving as theoretical values.

[2] The mixture of [1] was tableted to obtain an core tablet. During tableting, to confirm powder adhesion to the punch over time, tableting was stopped at 5, 10, and 20 minutes after the start of tableting, and evaluation was conducted using a colorimeter on the punch. A weight ratio of the phosphate co-crystal of Compound A in the core tablet is 50% converted to Compound A.

The results of the production are shown in Table 2.

[Table 2]

| | | Condition 2-1 | Condition 2-2 | Condition 2-3 | Condition 2-4 |
|---|---|---|---|---|---|
| Weight ratio of Compound A phosphate co-crystal in core tablet (%) | | 57.25 | 57.25 | 57.25 | 57.25 |
| (1) : (2)<br>(1) Total weight of Compound A phosphate co-crystal in powder<br>(2) Total weight of Compound A phosphate co-crystal in binding solution | | 10:0 | 7:3 | 5:5 | 3:7 |
| Median particle size of granulated product (μm) | | 126 | 99 | 146 | 242 |
| Particle size distribution of granulated product (%) | 710 μm on | 0.0 | 0.1 | 0.0 | 0.2 |
| | 500 μm on | 7.2 | 4.2 | 0.7 | 19.3 |
| | 355 μm on | 7.9 | 3.9 | 3.1 | 14.4 |
| | 250 μm on | 9.0 | 3.5 | 11.1 | 14.8 |
| | 180 μm on | 9.4 | 4.7 | 20.6 | 11.1 |
| | 150 μm on | 6.4 | 4.5 | 12.3 | 4.3 |
| | 106 μm on | 18.7 | 22.6 | 24.5 | 8.1 |
| | 75 μm on | 19.5 | 27.6 | 13.9 | 8.6 |
| | 75 μm pass | 21.9 | 28.9 | 13.9 | 19.1 |
| Bulk density of mixed granules (g/mL) | | 0.48 | 0.43 | 0.44 | 0.49 |
| Tapped density of mixed granules (g/mL) | | *0.55* | 0.54 | 0.49 | 0.57 |
| Hausner ratio of mixed granules | | 1.145 | 1.256 | 1.114 | 1.163 |
| Minimum orifice diameter of mixed granules (mm) | | 16 | 16 | 3.15 | 4 |

In all Condition 2-1 to 2-4, the flowability during granulation was good, and no adhesion to the container was observed.

The change over time in the punch measured using a colorimeter is shown in Fig. 1.

The color difference measured by the colorimeter is expressed as ΔE. When the ΔE value of the punch color before tableting is set to 0, as the powder adheres to the punch during tableting, the color changes from the initial state, and thus, the ΔE value increases.

As described above, the ratio of the amount of the phosphate co-crystal of Compound A charged into the fluidized bed granulator to the amount dispersed in the binding solution was varied, and granulation was performed by fluidized bed granulation, and the results are as shown in Fig. 1. That is, for the formulation systems of Condition 2-3 or 2-4, where the weight ratio of the phosphate co-crystal of Compound A charged into the fluidized bed granulator to the phosphate co-crystal of Compound A dispersed in the binding solution was set to 5:5 or 3:7, the ΔE values were confirmed to be smaller compared to the formulation systems of Condition 2-1 or 2-2 where the ratio was 10:0 or 7:3. That is, it was confirmed that powder adhesion to the punch was reduced by performing granulation by dispersing a portion of the phosphate co-crystal of Compound A in the binding solution as in Condition 2-3 or 2-4. This suggests that the degree of sticking is improved by performing granulation by dispersing a portion of the phosphate co-crystal of Compound A in the binding solution. Measurement conditions for the colorimeter are shown in Table 3.

[Table 3]

| Equipment used | SE-6000 | Nippon Denshoku Kogyo Co., Ltd. |
|---|---|---|

(continued)

| Measurement method | The upper punch was removed at an arbitrary timing after the start of tableting, and the average of the color difference values measured three times on the powder-contacting part of one punch was obtained.<br>When measuring the punch three times repeatedly, the punch was rotated by 120° for each measurement. |
|---|---|

[Example 3]

A tablet was obtained by performing fluidized bed granulation using a phosphate co-crystal of Compound A under the following conditions.

Condition 3-1: The weight ratio of the phosphate co-crystal of Compound A charged as powder into the fluidized bed granulator to the phosphate co-crystal of Compound A added to the binding solution was 0:10.

A preparation step of the binding solution was carried out according to the following procedure.

[1] 27 g of hydroxypropyl cellulose was gradually added to 473 g of purified water and stirred to dissolve.

[2] The entire suspension prepared in [1] was sieved through a sieve, and it was visually confirmed that there were no undissolved residues of hydroxypropyl cellulose, and a binding solution was obtained.

[3] 343.5 g of phosphate co-crystal of Compound A was gradually added to the aqueous solution of [2] while stirring, and the mixture was stirred and dispersed for at least 20 minutes.

[4] The entire suspension prepared in [3] was sieved through a sieve, and it was visually confirmed that there were no undissolved residues of hydroxypropyl cellulose or agglomerates of phosphate co-crystal of Compound A added when necessary.

[5] 100 g of purified water was added to the SUS container used in [3], any adhering drug substance was washed in, and the mixture was sieved through a sieve to obtain a binding solution.

Granulation and drying were carried out according to the following procedure.

[1] About 421.5 g of lactose hydrate and 30 g of carmellose calcium were charged in this order into a fluidized bed granulator.

[2] After preheating and mixing with an inlet air temperature set to 70 °C, the binding solution spray rate was adjusted to the target liquid rate and granulation was performed.

[3] After spraying the entire amount of the binding liquid, drying was performed at an inlet air temperature of 80 °C until the product temperature reached about 35 °C or higher, and a granulated product was obtained.

Mixing and tableting were carried out according to the following procedure.

[1] 45 g of carmellose calcium, 18 g of magnesium stearate, and the granulated product were mixed 100 times by bag mixing to obtain mixed granules. The amounts of carmellose calcium and magnesium stearate used in the present step were adjusted according to an actual yield of granulated product, with the above values serving as theoretical values.

[2] The mixture of [1] was tableted to obtain an core tablet. A weight ratio of the phosphate co-crystal of Compound A in the core tablet is 33.3% converted to Compound A.

The results of the production are shown in Table 4.

[Table 4]

| | Condition 3-1 |
|---|---|
| Weight ratio of Compound A phosphate co-crystal in core tablet (%) | 38.2 |

(continued)

|  | Condition 3-1 |
| --- | --- |
| (1) : (2) <br> (1) Total weight of Compound A phosphate co-crystal in powder <br> (2) Total weight of Compound A phosphate co-crystal in binding solution | 0:10 |
| Median particle size of granulated product (µm) | 305.4 |
| Particle size distribution of granulated product (%) | 710 µm on | 0.1 |
|  | 500 µm on | 5.1 |
|  | 355 µm on | 28.0 |
|  | 250 µm on | 35.6 |
|  | 180 µm on | 16.6 |
|  | 150 µm on | 3.8 |
|  | 106 µm on | 5.5 |
|  | 75 µm on | 2.8 |
|  | 75 µm pass | 2.7 |
| Bulk density of granulated product (g/mL) | 0.39 |
| Tapped density of granulated product (g/mL) | 0.41 |
| Hausner ratio of granulated product | 1.051 |
| Minimum orifice diameter of granulated product (mm) | 3.15 |

The flowability during granulation was good, no adhesion to the container was observed, and no sticking during tableting was observed.

[Example 4]

A tablet was obtained by performing fluidized bed granulation using a phosphate co-crystal of Compound A under the following conditions.

Condition 4-1: The weight ratio of the phosphate co-crystal of Compound A charged as powder into the fluidized bed granulator to the phosphate co-crystal of Compound A added to the binding solution was 0:10.

A preparation step of the binding solution was carried out according to the following procedure.

[1] 18 g of hydroxypropyl cellulose was gradually added to 282 g of purified water and stirred to dissolve.

[2] The entire suspension prepared in [1] was sieved through a sieve, and it was visually confirmed that there were no undissolved residues of hydroxypropyl cellulose, and a binding solution was obtained.

[3] 343.5 g of phosphate co-crystal of Compound A was gradually added to the binding solution of [2] while stirring, and the mixture was stirred and dispersed for at least 20 minutes.

[4] The entire suspension prepared in [3] was sieved through a sieve, and it was visually confirmed that there were no undissolved residues of hydroxypropyl cellulose or agglomerates of phosphate co-crystal of Compound A added when necessary.

[5] 100 g of purified water was added to the SUS container used in [3], any adhering drug substance was washed in, and the mixture was sieved through a sieve to obtain a binding solution.

Granulation and drying were carried out according to the following procedure.

[1] About 166.5 g of lactose hydrate and 30 g of carmellose calcium were charged in this order into a fluidized bed granulator.

[2] After preheating and mixing with an inlet air temperature set to 70 °C, the binding solution spray rate was adjusted to the target liquid rate and granulation was performed.

[3] After spraying the entire amount of the binding solution, drying was performed at an inlet air temperature of 80 °C until the product temperature reached about 35 °C or higher, and a granulated product was obtained.

Mixing and tableting were carried out according to the following procedure.

[1] 30 g of carmellose calcium, 12 g of magnesium stearate, and the granulated product were mixed 100 times by bag mixing to obtain mixed granules. The amounts of carmellose calcium and magnesium stearate used in the present step were adjusted according to an actual yield of granulated product, with the above values serving as theoretical values.
[2] The mixture of [1] was tableted to obtain an core tablet. A weight ratio of the phosphate co-crystal of Compound A in the core tablet is 50% converted to Compound A.

The results of the production are shown in Table 5.

[Table 5]

| | | Condition 1 |
|---|---|---|
| Weight ratio of Compound A phosphate co-crystal in core tablet (%) | | 57.25 |
| (1) : (2)<br>(1) Total weight of Compound A phosphate co-crystal in powder<br>(2) Total weight of Compound A phosphate co-crystal in binding solution | | 0:10 |
| Median particle size of granulated product ($\mu$m) | | 363 |
| Particle size distribution of granulated product (%) | 710 $\mu$m on | 0.1 |
| | 500 $\mu$m on | 5.1 |
| | 355 $\mu$m on | 28.0 |
| | 250 $\mu$m on | 35.6 |
| | 180 $\mu$m on | 16.6 |
| | 150 $\mu$m on | 3.8 |
| | 106 $\mu$m on | 5.5 |
| | 75 $\mu$m on | 2.8 |
| | 75 $\mu$m pass | 2.7 |
| Bulk density of granulated product (g/mL) | | 0.36 |
| Tapped density of granulated product (g/mL) | | 0.38 |
| Hausner ratio of granulated product | | 1.056 |
| Minimum orifice diameter of granulated product (mm) | | 3.15 |

The flowability during granulation was good, and no sticking during tableting was observed.

[Example 5]

The phosphate co-crystal of Compound A was entirely charged into a fluidized bed granulator, and fluidized bed granulation was performed as follows to obtain tablets. One granulation step was defined as one batch, and three batches obtained by repeating production three times were mixed together in a mixing step. Further, one mixing step was defined as one lot, and subsequent tableting and coating steps were carried out. (1 lot = 3 batches)

A preparation step of the binding solution was carried out according to the following procedure. (Description of 1 batch)

[1] 37.80 g of hydroxypropyl cellulose was gradually added to 434.7 g of purified water and stirred to dissolve.

Considering an amount of loss during the step, the amount charged was 150% of a theoretical amount.

[2] The entire suspension prepared in [1] was sieved through a sieve, and it was visually confirmed that there were no undissolved residues of hydroxypropyl cellulose.

Granulation and drying were carried out according to the following procedure. (Description of 1 batch)

[1] A portion of 401.8 g of lactose hydrate was added to a bag containing 320.6 g of phosphate co-crystal of Compound A, and mixed 50 times, and then the mixture was sieved through a sieve and charged into a granulator. After that, the remaining lactose hydrate was charged into the granulator.

[2] The binding solution spray rate was adjusted to the target liquid rate and granulation was performed.

[3] After spraying the entire amount of the binding solution, drying was performed at an intake temperature of 80 °C until the product temperature reached 45 °C, and a granulated product was obtained.

Mixing and tableting were carried out according to the following procedure. (Description of 1 lot)

[1] 252.0 g of low-substituted hydroxypropyl cellulose, 25.20 g of magnesium stearate, and the granulated product were charged into a mixer and mixed at a mixer rotation speed of 36 $min^1$ to obtain mixed granules. The amounts of low-substituted hydroxypropylcellulose and magnesium stearate used in the present step were adjusted according to an actual yield of the granulated product, with the above being theoretical values.

[2] The mixture of [1] was tableted to obtain 4 lots of core tablets. A weight ratio of the phosphate co-crystal of Compound A in the core tablet is 33.3% converted to Compound A.

A coating liquid step and coating step were carried out according to the following procedure. (Description of 1 lot)

[1] A coating ingredient was gradually added to purified water while stirring such that a solid content concentration reaches 10 w/w%, and the mixture was stirred for at least 45 minutes.

[2] The mixture was sieved through a sieve to obtain a coating liquid.

[3] The tablet was coated using an aeration coating system.

The results of the production are shown in Table 6.

[Table 6]

|  |  | Lot 1 | Lot 2 | Lot 3 | Lot 4 |
|---|---|---|---|---|---|
| Weight ratio of Compound A phosphate co-crystal in core tablet (%) |  | 38.2 | 38.2 | 38.2 | 38.2 |
| (1) : (2) <br> (1) Total weight of Compound A phosphate co-crystal in powder <br> (2) Total weight of Compound A phosphate co-crystal in binding solution |  | 10:0 | 10:0 | 10:0 | 10:0 |
| Median particle size of mixed granules (μm) |  | 83 | 123 | 86 | 74 |
| Particle size distribution of mixed granules (%) | 710 μm on | 0.1 | 0.1 | 0.1 | 0.0 |
|  | 500 μm on | 0.3 | 16.0 | 3.3 | 0.4 |
|  | 355 μm on | 0.5 | 10.4 | 4.3 | 0.4 |
|  | 250 μm on | 1.3 | 6.8 | 4.1 | 0.5 |
|  | 180 μm on | 4.4 | 5.4 | 4.0 | 1.5 |
|  | 150 μm on | 4.7 | 3.6 | 3.5 | 2.7 |
|  | 106 μm on | 20.5 | 12.7 | 15.8 | 17.2 |

(continued)

|  |  | Lot 1 | Lot 2 | Lot 3 | Lot 4 |
|---|---|---|---|---|---|
|  | 75 μm on | 24.3 | 15.2 | 23.3 | 26.5 |
|  | 75 μm pass | 43.9 | 29.9 | 41.5 | 50.8 |
| Minimum orifice diameter of mixed granules (mm) | | 3.15 | 3.15 | 8 | 3.15 |
| Bulk density of mixed granules (g/mL) | | 0.46 | 0.51 | 0.49 | 0.44 |
| Rate of tablets with defective appearance in sampled products (%) | | 1.5 | 14.2 | 100 | 5.0 |

In the present example, to produce a pharmaceutical composition in which the content of the phosphate co-crystal of Compound A in a core tablet is 30% by weight converted to Compound A, the phosphate co-crystal of Compound A was entirely charged into the granulator, and granulation was performed by fluid bed granulation, followed by mixing, tableting, and coating. When multiple lots were produced using the same formulation and under the same condition, tablets with poor appearance due to sticking during the tableting step were observed in all lots. However, the flowability during granulation was good, and no adhesion to the container was observed.

[Example 6]

A tablet was obtained by performing fluidized bed granulation using a phosphate co-crystal of Compound A under the following conditions.
Condition 6-1: The weight ratio of the phosphate co-crystal of Compound A charged as powder into the fluidized bed granulator to the phosphate co-crystal of Compound A added to the binding solution was 7:3.
A preparation step of the binding solution was carried out according to the following procedure.

[1] 36 g of hydroxypropyl cellulose was gradually added to 584 g of purified water and stirred to dissolve.

[2] The entire suspension prepared in [1] was sieved through a sieve, and it was visually confirmed that there were no undissolved residues of hydroxypropyl cellulose, and a binding solution was obtained.

[3] When necessary, 137.4 g of phosphate co-crystal of Compound A was gradually added to the aqueous solution of [2] while stirring, and the mixture was stirred and dispersed for at least 20 minutes.

[4] The entire suspension prepared in [3] was sieved through a sieve, and it was visually confirmed that there were no undissolved residues of hydroxypropyl cellulose or agglomerates of the added phosphate co-crystal of Compound A.

[5] 100 g of purified water was added to the SUS container used in [3], any adhering drug substance was washed in, and the mixture was sieved through a sieve to obtain a binding solution.

Granulation and drying were carried out according to the following procedure.

[1] 602.0 g of lactose hydrate, 40 g of carmellose calcium, and 320.6 g of phosphate co-crystal of Compound A were charged in this order into a fluidized bed granulator.

[2] After preheating and mixing with an inlet air temperature set to 70 °C, the binding solution spray rate was adjusted to the target liquid rate and granulation was performed.

[3] After spraying the entire amount of the binding solution, drying was performed at an inlet air temperature of 80 °C until the product temperature reached about 35 °C or higher, and a granulated product was obtained.

Mixing and tableting were carried out according to the following procedure.

[1] 29.7 g of carmellose calcium, 18 g of magnesium stearate, and the granulated product were mixed 50 times by bag mixing to obtain mixed granules. The amounts of carmellose calcium and magnesium stearate used in the present step were adjusted according to an actual yield of granulated product, with the above values serving as theoretical values.
[2] The mixture of [1] was tableted to obtain an core tablet. During tableting, to confirm powder adhesion to the punch over time, tableting was stopped at 5, 10, and 20 minutes after the start of tableting, and evaluation was conducted using a colorimeter on the punch. A weight ratio of the phosphate co-crystal of Compound A in the core tablet is 33% converted to Compound A.

The results of the production are shown in Table 7.

[Table 7]

| | | | Condition 6-1 |
|---|---|---|---|
| Weight ratio of Compound A phosphate co-crystal in core tablet (%) | | | 38.17 |
| (1) : (2)<br>(1) Total weight of Compound A phosphate co-crystal in powder<br>(2) Total weight of Compound A phosphate co-crystal in binding solution | | | 7:3 |
| Average particle size of granulated product (μm) | | | 121.0 |
| Particle size distribution of granulated product (%) | | 710 μm on | 0.3 |
| | | 500 μm on | 10.0 |
| | | 355 μm on | 11.9 |
| | | 250 μm on | 8.9 |
| | | 180 μm on | 8.6 |
| | | 150 μm on | 3.9 |
| | | 106 μm on | 9.9 |
| | | 75 μm on | 13.5 |
| | | 75 μm pass | 33.1 |
| Bulk density of granulated granules (g/mL) | | | 0.55 |
| Minimum orifice diameter of granulated granules (mm) | | | 25 |

Also in Condition 6-1, the flowability during granulation was good, and no adhesion to the container was observed.

The change over time in the punch measured using a colorimeter is shown in Fig. 2.

As described above, when the content of the phosphate co-crystal of Compound A per tablet was 33% by weight converted Compound A, and the weight ratio of the phosphate co-crystal of Compound A charged into the fluidized bed granulator to that dispersed in the binding solution was set at 70:30, and granulation was performed by fluidized bed granulation, the results are as shown in Fig. 2. The ΔE values were comparable to those of the formulation systems of Condition 2-3 or 2-4, which are formulations showing improved degree of sticking as shown in Fig. 1. Measurement conditions for the colorimeter are shown in Table 3.

[Example 7]

A tablet was obtained by performing fluidized bed granulation using a phosphate co-crystal of Compound A under the following conditions.

Condition 7-1: The weight ratio of the phosphate co-crystal of Compound A charged as powder into the fluidized bed granulator to the phosphate co-crystal of Compound A added to the binding solution was 3:7.

A preparation step of the binding solution was carried out according to the following procedure.

[1] 37.2 g of hydroxypropyl cellulose was gradually added to 606.8 g of purified water and stirred to dissolve.

[2] The entire suspension prepared in [1] was sieved through a sieve, and it was visually confirmed that there were no undissolved residues of hydroxypropyl cellulose, and a binding solution was obtained.

[3] When necessary, 320.6 g of phosphate co-crystal of Compound A was gradually added to the aqueous solution of [2] while stirring, and the mixture was stirred and dispersed for at least 20 minutes.

[4] The entire suspension prepared in [3] was sieved through a sieve, and it was visually confirmed that there were no undissolved residues of hydroxypropyl cellulose or agglomerates of the added phosphate co-crystal of Compound A.

[5] 100 g of purified water was added to the SUS container used in [3], any adhering drug substance was washed in, and the mixture was sieved through a sieve to obtain a binding solution.

Granulation and drying were carried out according to the following procedure.

[1] 64.4 g of lactose hydrate, 24.0 g of carmellose calcium, and 137.4 g of phosphate co-crystal of Compound A were charged in this order into a fluidized bed granulation dryer.

[2] After preheating and mixing with an inlet air temperature set to 70 °C, the binding solution spray rate was adjusted to the target liquid rate and granulation was performed.

[3] After spraying the entire amount of the binding solution, drying was performed at an inlet air temperature of 80 °C until the product temperature reached about 35 °C or higher, and a granulated product was obtained.

Mixing and tableting were carried out according to the following procedure.

[1] 19.8 g of carmellose calcium, 10.4 g of magnesium stearate, and the granulated product were mixed 50 times by bag mixing to obtain mixed granules. The amounts of carmellose calcium and magnesium stearate used in the present step were adjusted according to an actual yield of granulated product, with the above values serving as theoretical values.
[2] The mixture of [1] was tableted to obtain an core tablet. During tableting, to confirm powder adhesion to the punch over time, tableting was stopped at 5, 10, and 20 minutes after the start of tableting, and evaluation was conducted using a colorimeter on the punch. A weight ratio of the phosphate co-crystal of Compound A in the core tablet is 65% converted to Compound A.

The results of the production are shown in Table 8.

[Table 8]

| | | Condition 7-1 |
|---|---|---|
| Weight ratio of Compound A phosphate co-crystal in core tablet (%) | | 73.87 |
| (1) : (2)<br>(1) Total weight of Compound A phosphate co-crystal in powder<br>(2) Total weight of Compound A phosphate co-crystal in binding solution | | 3:7 |
| Average particle size of granulated product ($\mu$m) | | 102.0 |
| Particle size distribution of granulated product (%) | 710 $\mu$m on | 0.0 |
| | 500 $\mu$m on | 10.4 |
| | 355 $\mu$m on | 9.9 |
| | 250 $\mu$m on | 6.7 |
| | 180 $\mu$m on | 6.5 |
| | 150 $\mu$m on | 3.0 |
| | 106 $\mu$m on | 11.2 |
| | 75 $\mu$m on | 17.2 |
| | 75 $\mu$m pass | 35.1 |
| Bulk density of granulated granules (g/mL) | | 0.47 |
| Minimum orifice diameter of granulated granules (mm) | | 25 |

Also in Condition 7-1, the flowability during granulation was good, and no adhesion to the container was observed.

The change over time in the punch measured using a colorimeter is shown in Fig. 3.

As described above, when the content of the phosphate co-crystal of Compound A per tablet was 65% by weight converted Compound A, and the weight ratio of the phosphate co-crystal of Compound A charged into the fluidized bed granulator to that dispersed in the binding solution was set at 30:70, and granulation was performed by fluidized bed granulation, the results are as shown in Fig. 3. The ΔE values were comparable to those of the formulation systems of Condition 2-3 or 2-4, which are formulations showing improved degree of sticking as shown in Fig. 1. Measurement conditions for the colorimeter are shown in Table 3.

In this way, it was shown that a pharmaceutical composition in which the content of a phosphate co-crystals of Compound A in one tablet is 30% by weight or more converted to Compound A can be produced without any trouble during granulation. In particular, it was also confirmed that the poor appearance caused by sticking could be improved by performing granulation by dispersing a portion or all of the phosphate co-crystal of Compound A in the binding solution. Further, it was confirmed that a pharmaceutical composition under more severe conditions, in which the content of the phosphate co-crystal of Compound A in one tablet is 50% by weight converted to Compound A, can be produced, while avoiding all troubles in the production steps, such as sticking, poor flowability, and adhesion to the container, by performing granulation by dispersing a portion of the phosphate co-crystal of Compound A in the fluidized bed granulator and the rest in the binding solution, thereby improving productivity.

As shown above, the degree of sticking was improved under Condition 2-3 compared to Condition 2-2 in Example 2, and production was possible while avoiding the troubles in the production steps. Also under Condition 6-1 in Example 6 and Condition 7-1 in Example 7, production was possible while avoiding the troubles in the production steps.

For the four conditions, Condition 2-2, Condition 2-3, Condition 6-1, and Condition 7-1, the content (%) of Compound A or the like converted to Compound A based on the total weight of the pharmaceutical composition is plotted on the X-axis, and the weight ratio (%) of Compound A or the like in the binding solution during granulation is plotted on the Y-axis, and the relationship between the four points was calculated using the least squares method, and the result was Y = 1.23X - 15.79. In a range where the weight ratio (%) of Compound A or the like in the binding solution during granulation was above the value derived from this equation, production is possible while avoiding the troubles in the production steps, and the conditions such as Conditions 1-1 and 1-2 of Example 1, Condition 2-1 of Example 3, and Condition 4-1 of Example 4 were also included in this range, suggesting that this is a favorable range.

[Example 8]

The contact angle of the phosphate co-crystal of Compound A was measured according to the following procedure:

[1] The phosphate co-crystal of Compound A was compressed using a single punch tablet press to create a pellet with a flat surface.

[2] 5 μL of purified water was dropped onto the pellet, and an image was captured using a CCD camera 10 seconds after the drop.

[3] A static contact angle, which is an angle between the pellet and the dropped droplet, was measured from the image captured using the CCD camera.

The static contact angle of the phosphate co-crystal of Compound A measured according to the above procedure was 52.77° ± 2.72° (n=5). The average and standard deviation were calculated according to JIS Z 8401.

**Claims**

1. A pharmaceutical composition containing 1-{2-[(3S,4R)-1-{[(3R,4R)-1-cyclopentyl-3-fluoro-4-(4-methoxyphenyl) pyrrolidin-3-yl]carbonyl}-4-(methoxymethyl)pyrrolidin-3-yl]-5-(trifluoromethyl)phenyl}piperidine-4-carboxylic acid (Compound A) or a pharmaceutically acceptable salt or co-crystal thereof as an active ingredient, wherein a content of Compound A or a pharmaceutically acceptable salt or co-crystal thereof is 30% by weight or more converted to Compound A, based on a total weight of the pharmaceutical composition.

2. The pharmaceutical composition according to claim 1, further containing one or more selected from a disintegrant, a binder, and a filler.

3. The pharmaceutical composition according to claim 2, wherein a content of the disintegrant is 0.1% - 30% by weight, based on the total weight of the pharmaceutical composition.

4. The pharmaceutical composition according to claim 2 or 3, wherein the disintegrant is calcium carmellose or low-substituted hydroxypropyl cellulose.

5. The pharmaceutical composition according to any one of claims 2 - 4, wherein a content of the binder is 1% - 15% by weight, based on the total weight of the pharmaceutical composition.

6. The pharmaceutical composition according to any one of claims 2 - 5, wherein the binder is a water-soluble polymer.

7. The pharmaceutical composition according to claim 6, wherein the water-soluble polymer is hydroxypropyl cellulose.

8. The pharmaceutical composition according to any one of claims 2 - 7, wherein a content of the filler is 10% - 50% by weight, based on the total weight of the pharmaceutical composition.

9. The pharmaceutical composition according to any one of claims 2 - 8, wherein the filler is lactose hydrate.

10. The pharmaceutical composition according to any one of claims 1 - 9, further containing a lubricant.

11. The pharmaceutical composition according to claim 10, wherein a content of the lubricant is 0.1% - 5% by weight, based on the total weight of the pharmaceutical composition.

12. The pharmaceutical composition according to claim 10 or 11, wherein the lubricant is magnesium stearate.

13. The pharmaceutical composition according to any one of claims 1 - 12, wherein the content of Compound A or a pharmaceutically acceptable salt or co-crystal thereof is 50% by weight or more converted to Compound A, based on the total weight of the pharmaceutical composition.

14. The pharmaceutical composition according to any one of claims 1 - 13, being a granulated product of Compound A or a pharmaceutically acceptable salt or co-crystal thereof, a capsule containing the granulated product, or a tablet obtained by tableting the granulated product.

15. The solid pharmaceutical composition according to claim 14, wherein the granulated product is obtained by granulating a powder containing Compound A or a pharmaceutically acceptable salt or co-crystal thereof by fluidized bed granulation.

16. The pharmaceutical composition according to any one of claims 1 - 15, wherein the granulated product is composed of a portion that contains Compound A or a pharmaceutically acceptable salt or co-crystal thereof but substantially does not contain a binder, and a portion that contains Compound A or a pharmaceutically acceptable salt or co-crystal thereof and a binder.

17. The pharmaceutical composition according to any one of claims 1 - 16, wherein the granulated product is obtained by spraying a binding solution containing Compound A or a pharmaceutically acceptable salt or co-crystal thereof and a binder onto a powder that may contain Compound A or a pharmaceutically acceptable salt or co-crystal thereof.

18. The pharmaceutical composition according to any one of claims 1 - 17, wherein the granulated product is obtained by spraying a binding solution containing Compound A or a pharmaceutically acceptable salt or co-crystal thereof and a binder onto a powder containing Compound A or a pharmaceutically acceptable salt or co-crystal thereof.

19. The pharmaceutical composition according to claim 17 or 18, wherein a ratio of a total weight of Compound A or a pharmaceutically acceptable salt or co-crystal thereof in the powder to a total weight of Compound A or a pharmaceutically acceptable salt or co-crystal thereof in the binding solution is 70:30 - 0:100.

20. The pharmaceutical composition according to any one of claims 14 - 19, wherein a median particle size of the granulated product is 90 $\mu$m - 400 $\mu$m.

21. The pharmaceutical composition according to any one of claims 14 - 20, wherein an angle of repose of the granulated product is 50 degrees or less.

22. The pharmaceutical composition according to any one of claims 14 - 21, being a tablet obtained by tableting the granulated product.

23. The pharmaceutical composition according to claim 22, wherein the tablet has a hardness of 30 N or more.

24. The pharmaceutical composition according to claim 22 or 23, wherein when 6.5 g of the tablet is tested using a drum described in a friability test method for tablets in Japanese Pharmacopoeia, at a drum rotation speed of 25 rpm for 4 minutes, friability after correction for moisture absorption is 1% or less.

25. The pharmaceutical composition according to any one of claims 22 - 24, wherein the tablet shows an individual degradation product amount of 1% or less in a 6-month stability test under conditions of a temperature of 40 °C and a relative humidity of 75%.

26. The pharmaceutical composition according to any one of claims 1 - 25, wherein Compound A or a pharmaceutically acceptable salt or co-crystal thereof is a phosphate co-crystal of Compound A.

27. A method for producing the pharmaceutical composition according to any one of claims 1 - 26, comprising: a granulation step of obtaining a granulated product by fluidized bed granulation.

28. A method for producing the pharmaceutical composition according to any one of claims 1 - 26, comprising: a granulation step of obtaining a granulated product by spraying a binding solution containing Compound A or a pharmaceutically acceptable salt or co-crystal thereof and a binder onto a powder that may contain Compound A or a pharmaceutically acceptable salt or co-crystal thereof in a fluidized bed granulator.

29. A method for producing a pharmaceutical composition containing 1-{2-[(3S,4R)-1-{[(3R,4R)-1-cyclopentyl-3-fluoro-4-(4-methoxyphenyl)pyrrolidin-3-yl]carbonyl}   -4-(methoxymethyl)pyrrolidin-3-yl]-5-(trifluoromethyl)phenyl} piperidine-4-carboxylic acid (Compound A) or a pharmaceutically acceptable salt or co-crystal thereof as an active ingredient, the method comprising: a granulation step of obtaining a granulated product by spraying a binding solution containing Compound A or a pharmaceutically acceptable salt or co-crystal thereof and a binder onto a powder containing Compound A or a pharmaceutically acceptable salt or co-crystal thereof in a fluidized bed granulator.

30. A method for producing the pharmaceutical composition according to any one of claims 1 - 26, comprising: a granulation step of obtaining a granulated product by spraying a binding solution containing Compound A or a pharmaceutically acceptable salt or co-crystal thereof and a binder onto a powder containing Compound A or a pharmaceutically acceptable salt or co-crystal thereof in a fluidized bed granulator.

31. The method according to any one of claims 27 - 30, comprising: prior to the granulation step, a first mixing step of obtaining a mixture by mixing Compound A or a pharmaceutically acceptable salt or co-crystal thereof with an excipient and/or a disintegrant, or a first mixing step of obtaining a mixture by mixing only a filler and a disintegrant.

32. The method according to claim 31, comprising: prior to the granulation step, a first mixing step of obtaining a mixture by mixing Compound A or a pharmaceutically acceptable salt or co-crystal thereof with a filler and/or a disintegrant.

33. The method according to any one of claims 27 - 32, comprising: a second mixing step of further mixing a disintegrant and/or a lubricant with a granulated product obtained from the granulation step.

34. The method according to any one of claims 27 - 33, wherein a ratio of a total weight of Compound A or a pharmaceutically acceptable salt or co-crystal thereof in the powder used in the granulation step to a total weight of Compound A or a pharmaceutically acceptable salt or co-crystal thereof in the binding solution is 70:30 - 0: 100.

35. The method according to any one of claims 27 - 34, wherein a ratio of a total weight of Compound A or a pharmaceutically acceptable salt or co-crystal thereof in the powder used in the granulation step to a total weight of Compound A or a pharmaceutically acceptable salt or co-crystal thereof in the binding solution is 65:35 - 0:100.

36. The method according to any one of claims 27 - 35, wherein a ratio of a total weight of Compound A or a pharmaceutically acceptable salt or co-crystal thereof in the powder used in the granulation step to a total weight of Compound A or a pharmaceutically acceptable salt or co-crystal thereof in the binding solution is 50:50 - 30:70.

37. The method according to claim 36, wherein in the granulation step, a content of Compound A or a pharmaceutically acceptable salt or co-crystal thereof contained in a resulting solid pharmaceutical composition is 50% by weight or more converted to Compound A, based on the total weight of the pharmaceutical composition.

38. The method according to claim 37, wherein in the granulation step, a content of Compound A or a pharmaceutically acceptable salt or co-crystal thereof contained in a resulting pharmaceutical composition is 50% by weight converted to Compound A, based on the total weight of the pharmaceutical composition, and a ratio of a total weight of Compound A or a pharmaceutically acceptable salt or co-crystal thereof in the powder to a total weight of Compound A or a pharmaceutically acceptable salt or co-crystal thereof in the binding solution is 50:50.

39. The method according to claim 37, wherein in the granulation step, a content of Compound A or a pharmaceutically acceptable salt or co-crystal thereof contained in a resulting pharmaceutical composition is 50% by weight converted to Compound A, based on the total weight of the pharmaceutical composition, and a ratio of a total weight of Compound A or a pharmaceutically acceptable salt or co-crystal thereof in the powder to a total weight of Compound A or a pharmaceutically acceptable salt or co-crystal thereof in the binding solution is 30:70.

40. The method according to any one of claims 27 - 39, further comprising: a drying step of drying the granulated product.

41. The method according to any one of claims 27 - 40, further comprising: a tableting step of tableting a granulated product obtained from the second mixing step.

42. The method of claim 41, further comprising: a coating step of coating a tablet obtained from the tableting step.

43. A pharmaceutical composition containing 1-{2-[(3S,4R)-1-{[(3R,4R)-1-cyclopentyl-3-fluoro-4-(4-methoxyphenyl) pyrrolidin-3-yl]carbonyl}-4-(methoxymethyl)pyrrolidin-3-yl]-5-(trifluoromethyl)phenyl}piperidine-4-carboxylic acid (Compound A) or a pharmaceutically acceptable salt or co-crystal thereof as an active ingredient, the pharmaceutical composition being produced using a method comprising: a granulation step of obtaining a granulated product by spraying a binding solution containing Compound A or a pharmaceutically acceptable salt or co-crystal thereof and a binder onto a powder containing Compound A or a pharmaceutically acceptable salt or co-crystal thereof in a fluidized bed granulator.

Fig. 1

Fig. 2

Fig. 3

## INTERNATIONAL SEARCH REPORT

International application No.

**PCT/JP2023/028459**

### A. CLASSIFICATION OF SUBJECT MATTER

*A61K 31/454*(2006.01)i; *A61K 9/20*(2006.01)i; *A61K 9/48*(2006.01)i; *A61K 47/38*(2006.01)i; *A61P 1/04*(2006.01)i; *A61P 1/16*(2006.01)i; *A61P 7/00*(2006.01)i; *A61P 9/00*(2006.01)i; *A61P 9/10*(2006.01)i; *A61P 13/12*(2006.01)i; *A61P 17/00*(2006.01)i; *A61P 17/04*(2006.01)i; *A61P 17/14*(2006.01)i; *A61P 19/02*(2006.01)i; *A61P 19/06*(2006.01)i; *A61P 25/00*(2006.01)i; *A61P 25/04*(2006.01)i; *A61P 27/02*(2006.01)i; *A61P 29/00*(2006.01)i; *A61P 31/00*(2006.01)i; *A61P 31/04*(2006.01)i; *A61P 31/18*(2006.01)i; *A61P 35/00*(2006.01)i; *A61P 37/06*(2006.01)i; *A61P 43/00*(2006.01)i

FI: A61K31/454; A61K9/20; A61K9/48; A61K47/38; A61P43/00 111; A61P19/02; A61P29/00; A61P19/06; A61P1/04; A61P29/00 101; A61P17/00; A61P17/04; A61P37/06; A61P35/00; A61P7/00; A61P17/14; A61P25/04; A61P9/10; A61P25/00; A61P1/16; A61P13/12; A61P31/04; A61P31/18; A61P27/02; A61P9/00; A61P31/00

According to International Patent Classification (IPC) or to both national classification and IPC

### B. FIELDS SEARCHED

Minimum documentation searched (classification system followed by classification symbols)

A61K31/00-33/44; A61K9/00-9/72; A61K47/00-47/69; A61P1/00; A61P7/00; A61P9/00; A61P13/00; A61P17/00; A61P19/00; A61P25/00; A61P27/00; A61P29/00; A61P31/00; A61P35/00; A61P37/00; A61P43/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | WO 2021/251452 A1 (MITSUBISHI TANABE PHARMA CORP.) 16 December 2021 (2021-12-16) claims 1, 3, paragraphs [0016], [0097]-[0101], [0143]-[0147] | 1-18, 20-33, 40-43 |
| A | | 19, 34-39 |
| Y | JP 6959478 B1 (MITSUBISHI TANABE PHARMA CORP.) 02 November 2021 (2021-11-02) claim 1, paragraphs [0013], [0020]-[0023], [0040]-[0051] | 1-18, 20-33, 40-43 |
| A | | 19, 34-39 |
| Y | JP 2017-122056 A (OHARA PHARMACEUTICAL CO., LTD.) 13 July 2017 (2017-07-13) claims 1, 9, paragraphs [0002], [0007], [0009], [0010]-[0017] | 1-18, 20-33, 40-43 |

☑ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **02 October 2023** | **24 October 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

| International application No. |
| --- |
| **PCT/JP2023/028459** |

**C.    DOCUMENTS CONSIDERED TO BE RELEVANT**

| Category* | Citation of document, with indication, where appropriate, of the relevant passages | Relevant to claim No. |
| --- | --- | --- |
| A | WO 2020/138481 A1 (MITSUBISHI TANABE PHARMA CORP.) 02 July 2020 (2020-07-02)<br>  entire text | 1-43 |
| A | WO 2015/182723 A1 (MITSUBISHI TANABE PHARMA CORP.) 03 December 2015 (2015-12-03)<br>  entire text | 1-43 |

Form PCT/ISA/210 (second sheet) (January 2015)

## INTERNATIONAL SEARCH REPORT
### Information on patent family members

International application No.

**PCT/JP2023/028459**

| Patent document cited in search report | | | Publication date (day/month/year) | Patent family member(s) | | | Publication date (day/month/year) |
|---|---|---|---|---|---|---|---|
| WO | 2021/251452 | A1 | 16 December 2021 | EP 4166139 A1 claims 1, 3, paragraphs [0026], [0141]-[0145], [0189]-[0194] | | | |
| JP | 6959478 | B1 | 02 November 2021 | EP 4166140 A1 claim 1, paragraphs [0016], [0031]-[0033], [0053]-[0062] | | | |
| JP | 2017-122056 | A | 13 July 2017 | (Family: none) | | | |
| WO | 2020/138481 | A1 | 02 July 2020 | US 2022/0073497 A1 entire text | | | |
| WO | 2015/182723 | A1 | 03 December 2015 | US 2017/0190697 A1 entire text | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015182723 A

- WO 2020138481 A